# EUROPÄISCHE PATENTSCHRIFT

(11) **EP 3 111 930 B1**
(45) Veröffentlichungstag und Bekanntmachung des Hinweises auf die Patenterteilung: **18.04.2018**
(21) Anmeldenummer: 16177206.6
(22) Anmeldetag: 30.06.2016
(51) Int. Cl.: A61K 31/045, A61K 31/122, A61K 31/201, A61K 31/202, A61K 31/366, A61K 31/7076, A61K 36/38, A61K 36/67, A61P 25/24, A61K 31/7004, A61K 31/7016, A61K 36/41

(54) **ZUSAMMENSETZUNG ZUR BEHANDLUNG VON DEPRESSIONEN, BIPOLAREN STÖRUNGEN, REZIDIVIERENDEN DEPRESSIVEN STÖRUNGEN UND ANDEREN ANHALTENDEN AFFEKTIVEN STÖRUNGEN**
COMPOSITION FOR TREATING DEPRESSION, BIPOLAR DISORDERS, RECURRENT MEDICAL DEPRESSIVE DISORDERS AND OTHER LONG-TERM AFFECTIVE DISORDERS
COMPOSITION POUR LE TRAITEMENT DE DÉPRESSIONS, DE TROUBLES BIPOLAIRES, DE TROUBLES DÉPRESSIFS RÉCIDIVANTS ET D'AUTRES TROUBLES AFFECTIFS PERSISTANTS

(30) Priorität: 30.06.2015 EP 15001940
(43) Veröffentlichungstag der Anmeldung: 04.01.2017
(73) Patentinhaber: Bierwirth, Rolf, 6370 Reith bei Kitzbühel (AT)
(72) Erfinder: Mayer, Manfred, 84370 Kirchdorf am Inn (AT)
(74) Vertreter: Grünecker Patent- und Rechtsanwälte PartG mbB

(56) Entgegenhaltungen:
- WO-A1-98/03170
- WO-A1-2010/002406
- WO-A1-2010/133377
- KR-A-020110 134 961
- US-A1- 2012 177 732
- US-A1- 2014 348 883
- JAMES K. GLISSON ET AL: "Clinic at the Health Food Store? Employee Recommendations and Product Analysis", PHARMACOTHERAPY : THE JOURNAL OF HUMAN PHARMACOLOGY AND DRUG THERAPY, Bd. 23, Nr. 1, 1. Januar 2003 (2003-01-01) , Seiten 64-72, XP055233099, US ISSN: 0277-0008, DOI: 10.1592/phco.23.1.64.31912
- PELTON R: "BATTLING DEPRESSION, 'WORTS' AND ALL", AMERICAN DRUGGIST, XX, XX, Bd. 216, Nr. 9, 1. September 1999 (1999-09-01), Seite 48/49, XP008006878, ISSN: 0190-5279
- DATABASE WPI Week 200678 Thomson Scientific, London, GB; AN 2006-761347 XP002751766, & JP 2006 282509 A (UNIV TOKYO) 19. Oktober 2006 (2006-10-19)
- DATABASE WPI Week 201378 Thomson Scientific, London, GB; AN 2013-Q95822 XP002751767, & CN 103 110 102 A (XU B) 22. Mai 2013 (2013-05-22)
- Jerome Sarris: "Nutrients and herbal supplements for mental health", , 1. Januar 2014 (2014-01-01), XP055233060, Gefunden im Internet: URL:http://www.australianprescriber.com/ma gazine/37/3/article/1505.pdf [gefunden am 2015-12-02]
- FUGH-BERMAN A ET AL: "DIETARY SUPPLEMENTS AND NATURAL PRODUCTS AS PSYCHOTERAPEUTIC AGENTS", PSYCHOSOMATIC MEDICINE, ELSEVIER, NEW YORK, NY, US, Bd. 61, Nr. 5, 1. September 1999 (1999-09-01), Seiten 712-728, XP000890189, ISSN: 0033-3174
- JEROME SARRIS ET AL: "Major depressive disorder and nutritional medicine: a review of monotherapies and adjuvant treatments", NUTRITION REVIEWS, Bd. 67, Nr. 3, 1. März 2009 (2009-03-01), Seiten 125-131, XP055088451, ISSN: 0029-6643, DOI: 10.1111/j.1753-4887.2009.00180.x
- JEROME SARRIS ET AL: "Herbal medicine for depression, anxiety and insomnia: A review of psychopharmacology and clinical evidence", EUROPEAN NEUROPSYCHOPHARMACOLOGY, ELSEVIER SCIENCE PUBLISHERS BV, AMSTERDAM, NL, Bd. 21, Nr. 12, 6. April 2011 (2011-04-06) , Seiten 841-860, XP028119196, ISSN: 0924-977X, DOI: 10.1016/J.EURONEURO.2011.04.002 [gefunden am 2011-04-14]

## Beschreibung

Die vorliegende Erfindung bezieht sich auf eine pharmazeutischen Zusammensetzung zur Behandlung von Depressionen und anderen anhaltenden affektiven Störungen, umfassend (i) eine Hyperforin enthaltende Komponente, (ii) eine Kavapyrone enthaltende Komponente und/oder eine Rosavine enthaltende Komponente, (iii) eine Linalool und Linalylacetat enthaltende Komponente, (iv) eine S-Adenosylmethionin enthaltende Komponente und (v) eine Linolsäure und Linolensäure enthaltende Komponente, sowie optional zusätzlich einen Aminosäurekomplex.

### 1. Einleitung

Depressionen, auch "Depressive Störungen" genannt, gehören zu den häufigsten seelischen Erkrankungen. Die Erkrankten leiden neben einer gedrückten Stimmung und einer ausgeprägten Freud- und Interessenlosigkeit oftmals auch unter weiteren Beschwerden wie Konzentrationsstörungen, sozialem Rückzug, einer schnellen Ermüdbarkeit oder Schlafstörungen. Ein weiteres häufiges Symptom der Depression sind wiederholtes Grübeln und Gedankenkreisen. Weitere Symptome wie Appetitlosigkeit oder Frustessen, körperliche Beschwerden, Antriebslosigkeit, Tagesmüdigkeit sowie bei schweren Depressionen auch Suizidgedanken und Suizidhandlungen, können hinzu kommen. Zu den Störungen gehören akute, chronische und/oder episodisch auftretende Störungen wie depressive Verstimmungen, saisonale Stimmungsschwankungen bzw. Stimmungstiefs, saisonal abhängige Depressionen (Herbst-, Winterblues bzw. -depression (SAD)), Melancholie, sowie rezidivierende depressive beziehungsweise bipolare Störungen.

Im Laufe ihres Lebens erkranken etwa 12% der Menschen an einer Depression (Busch et al. Bundesgesundheitsbl. 2013, 56:733-739). Etwa 20% der Depressionen nehmen einen chronischen Verlauf. Dem Staat und der Wirtschaft in Deutschland entstehen jährlich Kosten bis zu 21,9 Milliarden Euro für die Behandlung sowie aufgrund von Fehlzeiten und verminderter Produktivität der betroffenen Mitarbeiter. Knapp 25 % aller Fehltage im Beruf gehen auf das Konto von Depressionen. Weiterhin ist die Dunkelziffer an nicht medizinisch erfassten Fällen von depressiven Störungen sehr hoch.

Es gibt verschiedene Therapieansätze zur Behandlung von Depressionen. Je nach Ausprägungsgrad der Depression werden die Durchführung einer Psychotherapie und/oder eine medikamentöse Behandlung empfohlen. In der medikamentösen Behandlung der Depressionen wird ab einem mittelschweren Ausprägungsgrad auch der alleinige oder ergänzende Einsatz von Antidepressiva angeraten. Folgende Wirkstoffgruppen für Antidepressiva finden Verwendung: nichtselektive Monoamin-Wiederaufnahmehemmer (NSMRI), selektive Serotonin-Wiederaufnahmehemmer (SSRI), selektive Serotonin-Noradrenalin-Wiederaufnahmehemmer (SSNRI), selektive Noradrenalin-Wiederaufnahmehemmer (SNRI) und Monoaminoxidase-Hemmer.

All diese Wirkstoffe besitzen unerwünschte Nebenwirkungen wie Tagesmüdigkeit, Gleichgewichtsstörungen, Mundtrockenheit, Gewichtszunahme, Obstipation, kardiale Erregungsleitungsstörungen, Senkung der Krampfschwelle, Verwirrtheit, Desorientierung und Delir (NSMRI) oder Unruhe, Schlaflosigkeit, Appetitlosigkeit, gastrointestinale Beschwerden wie Übelkeit, Erbrechen, Durchfall, Potenzprobleme, Orgasmusstörungen, Blutgerinnungsstörungen, vermehrtes Auftreten von Hämatomen, Epistaxis, Hypermenorrhoe, Angstsymptome sowie Erhöhung von Herzfrequenz und Blutdruck (SSRI und SSNRI), Schlafstörungen, Schwindel, Halluzinationen, Herzrhythmusstörungen, Tyramineffekt (Monoaminooxidase-Hemmer).

Bei den SSRI und den SSNRI besteht bei der Überdosierung ferner die Gefahr eines Serotonin-Syndroms, und ein plötzliches Absetzen der SSNRI kann wie auch bei den SSRI zu einen Absetzsyndrom führen. Ferner besteht bei der überwiegenden Anzahl von Antidepressiva die Gefahr einer Medikamentenabhängigkeit.

Den folgenden nicht rezeptpflichtigen Substanzen wurde ebenfalls eine positive Wirkung zugeschrieben: Echtes Johanniskraut (Hypericum perforatum), B-Vitamine, Mineralstoffe (Magnesium, Kalzium, Kalium), sowie Aminosäuren (besonders Tryptophan). Echtes Johanniskraut wirkt nicht bei einer einmaligen Einnahme. Wie chemische Antidepressiva auch muss das Heilmittel über mehrere Monate eingenommen werden, bis sich eine deutlich verbesserte Gemütslage abzeichnet. Zudem besitzen Präparate mit Johanniskraut ebenfalls Nebenwirkungen wie Manie, Kopfschmerzen, Müdigkeit und Sonnenempfindlichkeit. Besonders hellhäute Menschen können bei einer längeren Einnahme von Johanniskrautmitteln unter Hautirritationen, Rötungen und Jucken leiden. Auch während einer Schwangerschaft darf Johanniskraut auf keinen Fall eingenommen werden, da es eine stark abtreibende Wirkung besitzt.

Die Gruppe der B-Vitamine (Folsäure, Thiamin, Cobalmin, Pantothensäure, und Biotin) steht im Zusammenhang mit der Psyche eines Menschen; vielfach wird bei Blutuntersuchungen von depressiven Patienten ein Mangel der B-Vitamine nachgewiesen. Allerdings ist die generelle Wirksamkeit von B-Vitaminen bei depressiven Störungen nur unzureichend belegt.

Als Nahrungsergänzungsmittel mit antidepressiver Wirkung wird Eicosapentaensäure (EPA), eine mehrfach ungesättigte Fettsäure aus der Klasse der Omega-3-Fettsäuren, eine besondere Wirkung zur Stimmungsaufhellung und günstigen Einflussnahme auf Minderung von Depressionen zugeschrieben (Starris et al. Nutr. Rev. 2009 März; 67(3):125-31). Als Wirkungsmechanismus wird eine Interaktion von Fettsäure und dem Neurotransmitter Serotonin vermutet; ein Mangel an Serotonin korreliert häufig mit einem Mangel an Omega-3-Fettsäure, umgekehrt scheint die Gabe der Fettsäure zur Erhöhung des Serotoninspiegels zu führen. Es gibt keine Erkenntnisse, dass diese Mittel eine Depression nachhaltig therapieren.

WO 98/03170 offenbart ein Verfahren zur Verfügbarmachung von schwer wasserlöslichen Substanzen (z. B. aus Kava) und befasst sich hauptsächlich mit der Behandlung von Magenstörungen. Glisson, J. et al. (The Journal of Human Pharmacology and Drug Therapy, Vol. 23(1), (2003), S. 64-72) betrifft eine Studie zur Beurteilung von Empfehlungen von Drogeriemitarbeitern bezüglich Produkten, die sie für Depressionen empfehlen; Grundtenor des Artikels ist, dass die Empfehlungen der Drogeriemitarbeiter nicht hilfreich und zum Teil gefährlich sind und dass die Produkte z. T. in ihrem Gehalt an Wirkstoffen erheblich von den Packungsangaben abweichen. Pelton, R. (American Druggist.com, Vol. 216(9), Sept. 1999, S. 48-49) diskutiert die Wirksamkeit von u. a. Hypericin, SAMe und Kava in der Behandlung depressiver Störungen, warnt jedoch ausdrücklich, dass Johanniskraut bei schweren Depressionen nicht zu empfehlen ist. WO 2010/133377 offenbart eine Mischung aus alpha-Linolensäure, konjugierter Linolsäure und Ölsäure zur Erhöhung des Spiegels an essentiellen Fettsäuren im Körper. JP 2006 282509 offenbart eine Zusammensetzung zur Behandlung von postmenstrualen Stimmungsschwankungen; die Zusammensetzung enthält Linalylacetat als wirksamen Bestandteil und wird als Aromatherapie verabreicht. CN 103 110 102 betrifft ein Nahrungsergänzungsmittel zur Behandlung kognitiver Störungen, die durch Mangelernährung hervorgerufen werden, umfassend Pinienöl, Reisöl, Reiskeimöl, Linolsäure, Teesamen, Pecanöl und alpha-Linolensäure. KR 10201 1013 4961 betrifft eine Zusammensetzung zur (präventiven) Behandlung von Erkrankungen des zentralen Nervensystems, welche Linalool enthält. Sarris, J. (Australian Prescriber Vol. 31(3), June 2014, S. 90-93) diskutiert die Wirksamkeit von Nahrungszusätzen und Naturpräparaten bei verschiedenen geistigen Störungen und rät zur Zurückhaltung bei der Kombination von Kava mit anderen Antidepressiva. Fugh-Berman, A. et al. (Psychsomatic Medicine Vol. 61, 1999, S. 712-728) diskutiert die vorhandene Datenlage zur Behandlung von Depressionen und Angststörungen mit Pflanzen-basierten Wirkstoffen. Sarris, J. et al. (Nutrition Reviews, Vol. 67(3), 2009, S. 125-131) diskutiert die positive Korrelation der Verwendung von Omega-3-Fettsäuren, Folat, SAMe, Inositol und Aminosäuren als Additiva in der Behandlung von Depressionen mit klassischen Antidepressiva. Sarris, J., et al. (European Neuropsychopharmacology Vol. 21, Nr. 12, S. 841-860) gibt einen Überblick über die wesentlichen phytopharmakologischen Wirkstoffe, die - bis 2011 - in klinischen Studien bei Behandlungen gegen Depressionen getestet wurden. Die Autoren stellen zusammenfassend fest, dass ein umfassender Überblick bis dahin gefehlt hat und die Kräutermedizin in der Psychiatrie immer noch in den Kinderschuhen steckt. WO 2010/002406 beschreibt eine Erfindung, die mit einer Kombination von Extrakten aus Rhodiola rosea und einem oder mehreren Extrakten aus der Gruppe von Magnolia officinalis und Phillodendron amurense Störungen basierend auf "adrenal fatigue" und Insulinresistenz. US 2014/348883 offenbart eine Kombination aus Acetyl-L-carnitine, Vinpocetine, Hyperzine A, Alpha Lipoid acid, Biotin und Rhadiola als Nahrungsergänzungsmittel zur Verbesserung mentaler Leistungen und zur Behandlung von Stimmungsschwankungen. US 2012/177732 beschreibt die Verwendung natürlicher Inhaltsstoffe (u. a. Rhodiola rosea, Germanium oil, vinpocetine oder vincamine, phosphatidylcholine, anhydrous caffeine, Salix alba, NADH, Vanillin oder Peppermint oil) zur Verbesserung mentaler Zustände, wie der Konzentrationsfähigkeit.

Es besteht aufgrund der Prävalenz von depressiven Störungen und der signifikanten Nebenwirkungen medikamentöser Therapien weiterhin ein hoher Bedarf an wirksamen und nebenwirkungsfreien Alternativtherapien, die einfach in der Anwendung sind und allein oder unterstützend zu anderen Therapieformen eingesetzt werden können. Ferner besteht aufgrund der hohen Dunkelziffer ein Bedarf an sicheren (nebenwirkungsfreien) Therapieformen für Menschen, die keinen medizinischen Beistand zur Behandlung ihrer Probleme suchen und ansonsten zu anderen, nicht verlässlichen oder schädlichen Maßnahmen (Alkohol, Frustessen, etc.) greifen.

### 2. Zusammenfassung der Erfindung

Die vorliegende Aufgabe wird durch die vorliegende Erfindung gelöst, denn sie bietet eine wirksame, nebenwirkungsfreie und in der Anwendung sichere Möglichkeit zur Behandlung von depressiven Störungen, die allein oder in Kombination mit anderen Therapieformen eingesetzt werden kann. Für die vorliegende Zusammensetzung aus verschiedenen Komponenten wurde gefunden, dass sie schnell und erfolgreich in der Behandlung depressiver Störungen wirkt. Dabei ist für ein Erreichen des therapeutischen Effekts die Dosierung der einzelnen Komponenten wesentlich niedriger als die im Stand der Technik beschriebene wirksame Dosierung der jeweiligen Einzelkomponente allein. Dadurch wird das Risiko von Nebenwirkungen weiter minimiert.

Ein Aspekt der vorliegenden Erfindung ist eine Zusammensetzung, umfassend die Komponenten (i) bis (v) (i) eine Hyperforin enthaltende Komponente, (ii) eine Kavapyrone enthaltende Komponente oder eine Rosavine enthaltende Komponente, (iii) eine Linalool und Linalylacetat enthaltende Komponente, (iv) eine S-Adenosylmethionin enthaltende Komponente, (v) eine Linolsäure und Linolensäure enthaltende Komponente, und optional (vi) ein Aminosäurekomplex. Optional kann die Zusammensetzung ferner molekulare Mikronährstoffe und/oder *Mucuna pruriens* (z.B. Extrakt) enthalten. Die Zusammensetzung kann als pharmazeutische Zusammensetzung ausgeführt sein.

Ein weiterer Aspekt der vorliegenden Erfindung ist die Verwendung der erfindungsgemäßen (pharmazeutischen) Zusammensetzung in der Behandlung einer Depression.

### 3. Figurenbeschreibung

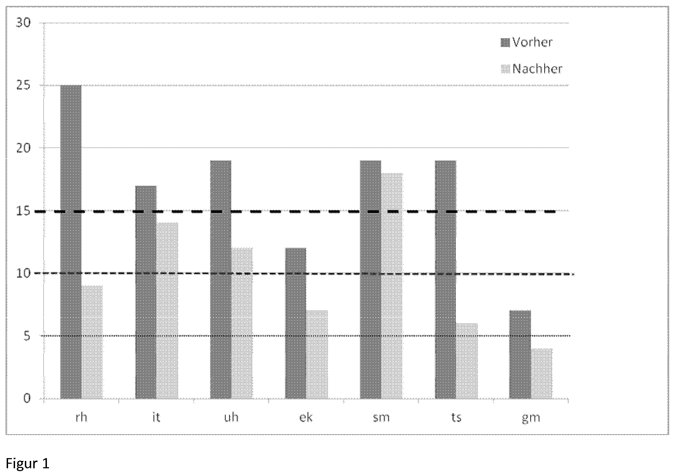

Figur 1 stellt die Ergebnisse der ersten klinischen Studie dar, wobei für jeden Patienten (Initialen in Kleinbuchstaben) der Skalensummenwert (Score) des PHQ-9 Depressionserhebungsbogens vor (dunkelgrau, links) und nach (hellgrau, rechts) der Behandlung aufgeführt sind. Die gestrichelte Line bei dem Wert 15 markiert den Übergang zur schweren depressiven Symptomatik (Scores von 15-27), die gestrichelte Linie bei dem Wert 10 markiert den Übergang zur mittelgradigen depressiven Symptomatik (Scores von 10-14), die Linie bei dem Wert 5 markiert den Übergang zur milden depressiven Symptomatik (Scores von 5-9); Werte <5 beschreiben eine nicht pathologische, minimale depressive Symptomatik.

### 4. Detaillierte Beschreibung der Erfindung

Ein Aspekt der vorliegenden Erfindung ist eine (pharmazeutische) Zusammensetzung, umfassend die Komponenten (1) bis (v) (i) eine Hyperforin enthaltende Komponente, (ii) eine Kavapyrone enthaltende Komponente oder eine Rosavine enthaltende Komponente, (iii) eine Linalool und Linalylacetat enthaltende Komponente, (iv) eine S-Adenosylmethionin enthaltende Komponente, (v) eine Linolsäure und Linolensäure enthaltende Komponente und optional (vi) ein Aminosäurekomplex. Optional enthält die Zusammensetzung ferner molekulare Mikronährstoffe. In einer Ausführungsform der vorliegenden Erfindung umfasst die pharmazeutische Zusammensetzung fünf der erfindungsgemäßen Komponenten (i) bis (v) und optional eine oder zwei der Komponenten (iv) und Mikronährstoffe.

### 4.1 Die Hyperforin enthaltende Komponente (i):

Der hierin verwendete Begriff "Hyperforin enthaltende Komponente" kann den reinen Stoff Hyperforin bezeichnen, eine Kombination des reinen Stoffs Hyperforin mit einem oder weiteren Stoffen, einen Pflanzenextrakt, der Hyperforin enthält, eine Kombination aus Pflanzenextrakten, die Hyperforin enthalten, die Kombination aus einem Pflanzenextrakt, der Hyperforin enthält, mit einem oder weiteren Stoffen, sowie die Kombination aus einer Kombination aus Pflanzenextrakten, die Hyperforin enthalten, mit einem oder weiteren Stoffen. Diese Stoffe können dem Fachmann bekannte Trägerstoffe oder Hilfsstoffe für die Herrichtung einer pharmazeutischen Zusammensetzung sein, insbesondere die erfindungsgemäßen mittelkettigen Triglyceride (MCT) und andere Öle, Wachse und Fette.

Hyperforin (IUPAC: (1*S*,5*S*,7*S*,8*R*)-4-hydroxy-8-methyl-3,5,7-tris(3-methylbut-2-enyl)-8-(4-methylpent-3-enyl)-1-(2-methylpropanoyl)-bicyclo[3.3.1]non-3-en-2,9-dion, CAS-Nummer 11079-53-1) hat die folgende Strukturformel (H1):

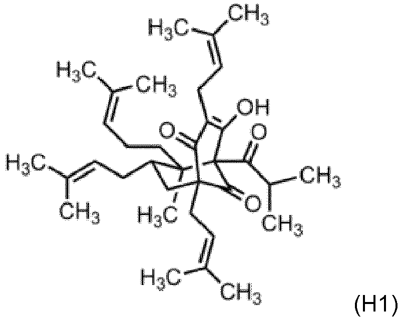

Adhyperforin, (IUPAC (1R,5S,6R,7S)-4-Hydroxy-6-methyl-5-(2-methylbutanoyl)-1,3,7-tris(3-methyl-2-buten-1-yl)-6-(4-methyl-3-penten-1-yl)bicyclo[3.3.1]non-3-ene-2,9-dion) hat die folgende Strukturformel (H2):

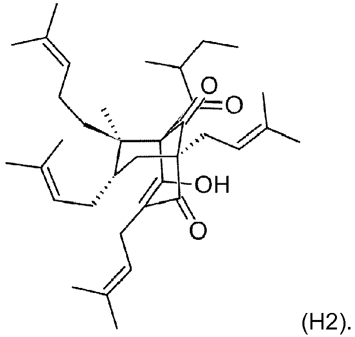

Erfindungsgemäß werden unter dem Begriff "Hyperforin" die Substanzen Hyperforin (H1) und Adhyperforin (H2) subsumiert, d.h. wenn von einem Hyperforin-Gehalt gesprochen wird, so kann sich dies auf den Anteil einer der beiden Substanzen (H1) oder (H2), sowie bevorzugt auf den Gehalt von (H1) und (H2) zusammengenommen beziehen. Alternativ wird letzteres auch als Gesamthyperforingehalt bezeichnet, oder es wird der Plural verwendet (Hyperforine).

In einer besonderen Ausführungsform der vorliegenden Erfindung umfasst die Hyperforin enthaltende Komponente der pharmazeutischen Zusammensetzung 15 bis 70 % (w/w) Hyperforine, bevorzugt 20-50% (w/w). Besonders bevorzugt sind hierbei 30-45 % (w/w) Hyperforine, oder etwa 35 % (w/w). Die Hyperforin-enthaltende Komponente kann 1-15% (w/w), bevorzugt 3-10% (w/w) Adhyperforin enthalten.

In einer bevorzugten Ausführungsform der vorliegenden Erfindung enthält oder ist die erfindungsgemäße Komponente (i) ein Pflanzenextrakt. In einer besonders bevorzugten Ausführungsform enthält oder ist die erfindungsgemäße Komponente (i) ein Pflanzenextrakt aus den Zweigspitzen oder Blüten des *Hypericum perforatum* (Echtes Johanniskraut) oder *Hypericum calycinum* (großkelchiges Johanniskraut). Ebenso kann erfindungsgemäß jeder andere Pflanzenextrakt verwendet werden, der einen erfindungsgemäßen Anteil an Hyperforin besitzt.

Die erfindungsgemäßen Pflanzenextrakte der Komponente (i) enthalten wenigstens 15, 20, 25, 30, 35, 40, 45 oder 50 % (w/w) Hyperforin. Besonders bevorzugt sind hierbei 30-45 % (w/w) Hyperforin.
In einer bevorzugten Ausführungsform der erfindungsgemäßen pharmazeutischen Zusammensetzung enthält oder besteht die Komponente (i) aus einem der kommerziell erhältlichen *Hypericum perforatum* Extrakten mit der CAS-Nr. 84082-80-4 oder der EINECS-Nr. 282-026-4. In einer weiteren bevorzugten Ausführungsform umfasst die erfindungsgemäße Komponente (i) Tocopherol (CAS-Nr. 59-02-9, EINECS-Nr. 200-412-2). In einer bevorzugten Ausführungsform ist der erfindungsgemäße Pflanzenextrakt der Komponente (i) der Johanniskrautextrakt der Firma Flavex mit der Typ Nummer 028.001.

In einer bevorzugten Ausführungsform der erfindungsgemäßen pharmazeutischen Zusammensetzung ist der Hypericin-Gehalt (IUPAC: 1,3,4,6,8,13-Hexahydroxy-10,11-dimethyl-phenanthro[1,10,9,8-opqra] perylen-7,14-dion) der Hyperforin enthaltenden Komponente (i) oder des erfindungsgemäßen Pflanzenextrakts kleiner als 1 % (w/w), vorzugsweise kleiner als 0,2 % (w/w), und besonders bevorzugt kleiner als 0,05 % (w/w). Besonders bevorzugt enthält die Hyperforin enthaltenden Komponente (i) oder der erfindungsgemäße Pflanzenextrakt kein, oder kein nachweisbares Hypericin.

Ferner kann die pharmazeutische Zusammensetzung in allen Ausführungsformen statt Hyperforin, oder zusätzlich zu Hyperforin, hyperforinanaloge Substanzen enthalten. Hyperforinanaloga sind z.B. Hyperforin-Tautomere, Aristoforin (H3), Hyperforin-trimethoxybenzoat (H4), Tetrahydrohyperforin, Octahydrohyperforin und/oder Hyperforin-nicotinat (H5).

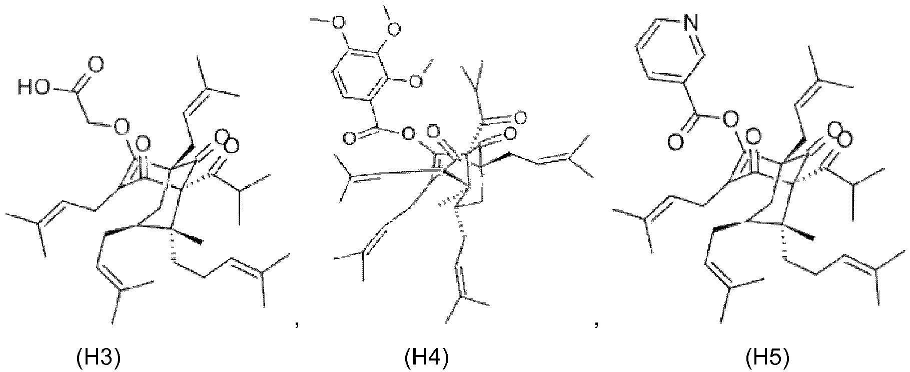

Herstellung: Bevorzugterweise wird die Hyperforin enthaltende Komponente durch Hochdruckextraktion mit natürlicher Quellkohlensäure (CO₂-Extraktion) hergestellt und enthält daher keine Lösungsmittelrückstände und keine Rückstände von anorganischen Salzen und Schwermetallen, sowie keine vermehrungsfähigen Keime. Der Extrakt ist vorzugsweise durch Zusatz von MCT-Öl standardisiert.

Die in Johanniskraut enthaltenen Wirkstoffe haben vielfältige Effekte auf das menschliche Nervensystem. Insbesondere das Hyperforin beeinflusst das Nervensystem in der Art, dass es zu einer Wiederherstellung des Gleichgewichts der Botenstoffe im Gehirn kommt. Die stimmungsaufhellende, angstlösende und antidepressive Wirkung von Johanniskraut beruht auf einer Erhöhung der Konzentration bestimmter Botenstoffe wie Dopamin, Serotonin und Noradrenalin, die bei depressiven Verstimmungen vermindert im Gehirn freigesetzt werden. Hyperforin hemmt die Wiederaufnahme dieser Stoffe in die Nervenendigung, wodurch sie schneller wieder zur Verfügung stehen bzw. dauerhaft wirken können. Bei dauerhafter Anwendung vermag Johanniskraut so den Nervenstoffwechsel zu regulieren.

### 4.2 Die Kavapyrone und/oder Rosavine enthaltende(n) Komponente(n) (ii):

Der hierin verwendete Begriff "Kavapyrone" (also der Plural) kann ein Kavapyron oder eine Mischung aus mindestens zwei verschiedenen Kavapyronen bezeichnen. Der hierin verwendete Begriff "Kavapyrone enthaltende Komponente" kann den reinen Stoff eines Kavapyrons, eine Mischung aus verschiedenen Kavapyronen (jeweils der reine Stoff), oder eine Kombination dieser mit einem oder weiteren Stoffen bezeichnen. Ebenso kann der Begriff einen Pflanzenextrakt oder eine Kombination aus Pflanzenextrakten bezeichnen, der oder die Kavapyrone enthalten. Ferner kann der Begriff einer Mischung solcher Pflanzenextrakte mit einem oder weiteren Stoffen bezeichnen. Diese Stoffe können dem Fachmann bekannte Trägerstoffe oder Hilfsstoffe für die Herrichtung einer pharmazeutischen Zusammensetzung sein, insbesondere die erfindungsgemäßen mittelkettigen Triglyceride (MCT) und andere Öle, Wachse und Fette. Analog hierzu kann eine "Rosavine enthaltende Komponente" den reinen Stoff Rosavin, eine Mischung aus verschiedenen Rosavinen (jeweils der reine Stoff) oder eine Kombination dieses Stoffes mit weiteren Stoffen, vorzugsweise Salidrosid, bezeichnen. Ebenso kann der Begriff Rosenwurzpulver, einen Pflanzenextrakt oder eine Kombination aus Pflanzenextrakten bezeichnen, der oder die Rosavin enthalten. Ferner kann der Begriff einer Mischung solcher Pflanzenextrakte mit einem oder weiteren Stoffen, wie Träger- oder Hilfsstoffe, wie oben für die Kavapyrone enthaltende Komponente beschrieben, bezeichnen.

In einer bevorzugten Ausführungsform der vorliegenden Erfindung enthält oder ist die erfindungsgemäße Komponente (ii) ein Pflanzenextrakt. In einer besonders bevorzugten Ausführungsform enthält oder ist die erfindungsgemäße Komponente (ii) ein Pflanzenextrakt aus der Wurzel des *Piper methysticum.* Ebenso kann erfindungsgemäß jeder andere Pflanzenextrakt verwendet werden, der einen erfindungsgemäßen Anteil an Kavapyronen besitzt. Ist oder enthält die Komponente (ii) eine Rosavine enthaltende Komponente, so ist sie vorzugsweise Rosenwurzpulver oder ein Extrakt aus Rosenwurz (*Rhodiola rosea*). Alternativ kann jeder andere Pflanzenextrakt verwendet werden, der einen erfindungsgemäßen Anteil an Rosavinen enthält.

Kavapyrone (auch Kavalactone genannt) sind substituierte, sechsgliedrige Lactone, sogenannte Styryl-alpha-Pyrone. Man unterscheidet zwei Gruppen, die Enolidpyrone und die Dienolidpyrone. Bei der ersten Gruppe enthält der Lactonring eine Doppelbindung, bei der zweiten sind zwei Doppelbindungen vorhanden. Allen Kavapyronen gemeinsam ist eine β-ständige Methoxygruppe im Lactonring. Kavapyrone der folgenden Strukturformeln (K1) - (K8) sind besonders bevorzugt: 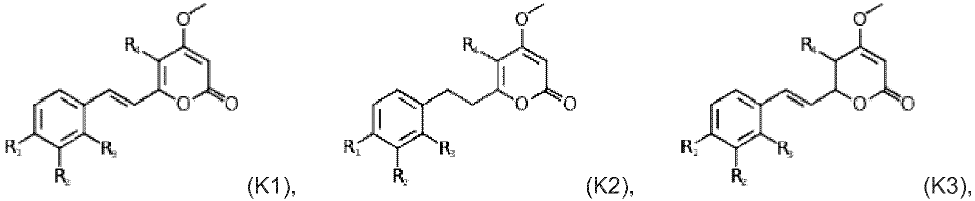 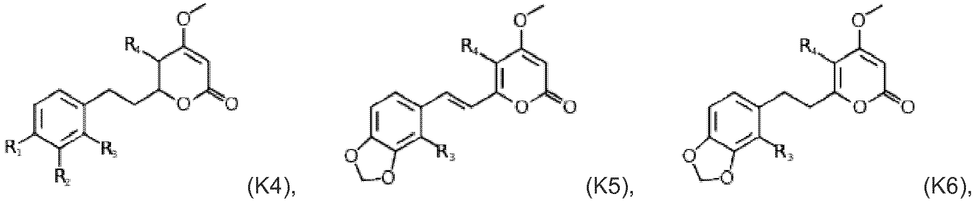 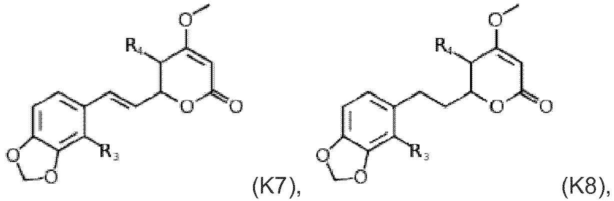 wobei R₁, R₂, R₃, und R₄ unabhängig voneinander ausgewählt sind aus der Gruppe, bestehend aus H, OH und OCH₃. Ausgewählte Kavapyrone sind:

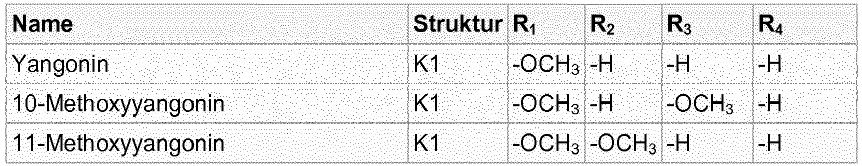

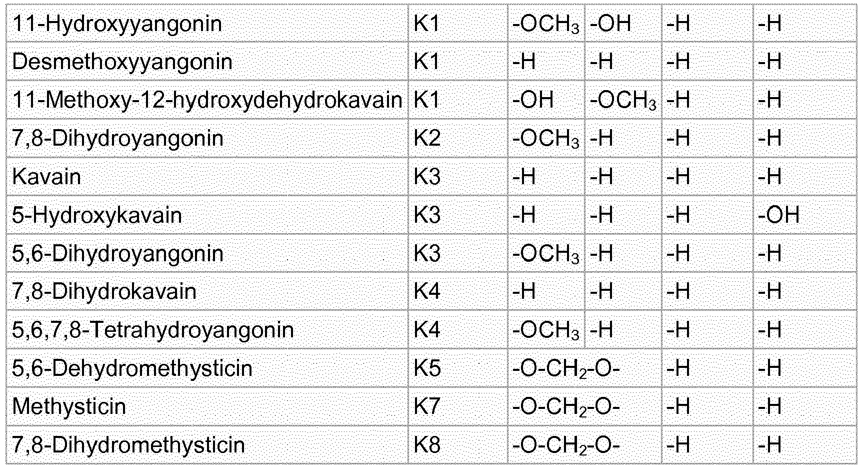

In einer besonders bevorzugten Ausführungsform der erfindungsgemäßen pharmazeutischen Zusammensetzung enthält die Komponente (ii) die Kavapyrone Kavain (z.B. D,L Kavain), Dihydrokavain (z.B. 7,8 Dihydrokavain), Methysticin, Dihydromethysticin (z.B. 7,8-Dihydromethysticin), Yangonin und Desmethoxyyangonin (z.B. 1,2-Desmethoxyyangonin):

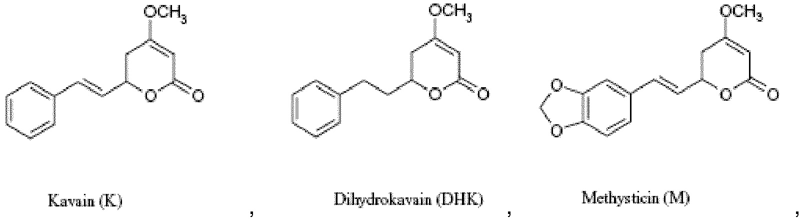

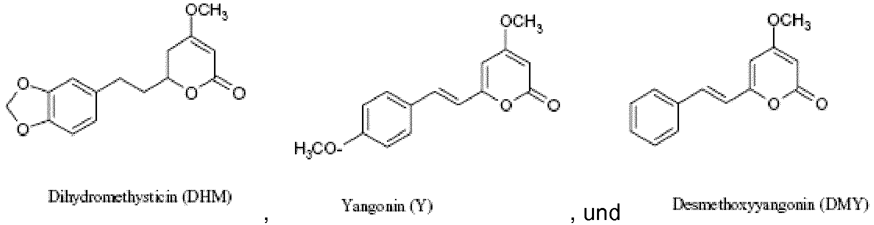

In einer besonders bevorzugten Ausführungsform enthält die Kavapyrone enthaltende Komponente (ii) ein, zwei, drei, vier, fünf oder sechs der o.g. Kavapyrone.

Die Kavapyrone enthaltende Komponente (ii) enthält bevorzugterweise wenigstens 30 % (w/w) Kavapyrone und besonders bevorzugt wenigstens 40 % (w/w), wenigstens 50 % (w/w) oder wenigstens 55 % (w/w) Kavapyrone. In einer bevorzugten Ausführungsform der vorliegenden Erfindung umfasst die Kavapyrone enthaltende Komponente der pharmazeutischen Zusammensetzung 15 bis 80 % (w/w) Kavapyrone, bevorzugt 50%-70% (w/w). Besonders bevorzugt sind hierbei 55-65% oder circa 60 % (w/w) Kavapyrone.

In einer besonders bevorzugten Ausführungsform ist der Pflanzenextrakt der Komponente (ii) der Kava-Kavawurzelextrakt der Firma Flavex mit der Typ Nummer 089.002. Optional kann die Kavapyrone enthaltende Komponente Wachse, Olivenöl und Polysorbat umfassen. Besonders bevorzugt umfasst die Kavapyrone enthaltende Komponente (ii) die folgenden Bestandteile: INCI-Name: Piper Methysticum Root Extract [CO2], CAS-Nr.: 84696-40-2 EG-Nr. (EINECS/ELINCS): 283-648-9, Olea Europaea Fruit Oil, CAS-Nr.: 8001-25-0 EG-Nr. (EINECS/ELINCS): 232-277-0, und Polysorbate 80, CAS-Nr.: 9005-65-6 und weist einen Gesamtgehalt an Kavapyronen von etwa 58 - 62 % auf mit den Kavapyronen Kavain, 7,8-Dihydrokavain, 7,8-Dihydromethysticin, Yangonin und 1,2-Desmethoxyyangonin.

Herstellung: Bevorzugterweise wird die Kavapyron enthaltende Komponente durch Hochdruckextraktion mit natürlicher Quellkohlensäure (CO₂-Extraktion) hergestellt und enthält daher keine Lösungsmittelrückstände und keine Rückstände von anorganischen Salzen und Schwermetallen sowie keine vermehrungsfähigen Keime. Der Extrakt wird vorzugsweise mit Olivenöl standardisiert und durch die Zugabe von Polysorbat homogenisiert.

Kavalactone (Kavapyrone) binden an verschiedene Nervenzellen und üben dabei eine gefühlsdämpfende Wirkung aus. Das führt zu beruhigenden, ruhigstellenden, schmerzlindernden und muskelentspannenden Effekten. Dazu kommen angstlösende und antidepressive Effekte. Die Wirksamkeit von Kava Kava bei Patienten mit Angst-, Spannungs- und Aufregungszuständen oder Depressionen wurde belegt. Allerdings muss das Präparat für eine längere Zeit eingenommen werden, die maximale Wirkung tritt nach 4 Wochen ein. Von vielen Ärzten wurde Kava Kava als pflanzliche Alternative zu den synthetischen angstlösenden Mitteln, wie Benzodiazepinen, eingesetzt; gegenüber den chemischen Mitteln hat Kava Kava den Vorteil, nicht abhängig zu machen, weder auf körperlicher noch auf seelischer Ebene. Auch Aspekte eines gesunden Schlafs werden durch die in Kava Kava enthaltenen Bestandteile unterstützt (zum Beispiel schnelleres Einschlafen, längerer Tiefschlaf). Dabei wird die Traumphase nicht beeinträchtigt, wie das bei chemischen Mitteln häufig geschieht und der "künstliche" Schlaf oft keine ausreichend erholsame Wirkung hat. Die Bestandteile der Kava Kava Komponente verursachen keine Beeinträchtigung der Aufmerksamkeit und Reaktionsbereitschaft. Im Gegenteil, es gibt Hinweise für eine erhöhte Aufmerksamkeit mit einer verbesserten Gedächtnisleistung und Verringerung der Reaktionszeit. Kava Kava hebt also die Stimmung und fördert die psychische Stabilität, ohne dabei müde zu machen oder die Koordination zu beeinflussen. Kava Kava Extrakt wirkt im Tierversuch lindernd auf Muskelkrämpfe, die durch verschiedene Ursachen ausgelöst wurden, inklusive epileptischen Zuständen. Vermutlich geht diese Eigenschaft auf eine generelle Hemmung der Erregbarkeit von Nervenzellen zurück. Die Lösung von Krämpfen betrifft nicht nur die Skelettmuskulatur, sondern auch die unbewusst gesteuerte Muskulatur wie die von Darm und Gebärmutter. Die Bestandteile von Kava Kava wirken zusätzlich als leichtes Schmerzmittel. Auf der menschlichen Zunge hat der Extrakt eine zumindest lokalanästhetische Wirkung.

Die Rosenwurz (*Rhodiola rosea*) ist ein stimulierendes und adaptogenes pflanzliches Heilmittel. Extrakte aus der Wurzel und dem Rhizom werden zur Linderung körperlicher und geistiger Symptome bei Stress und Überarbeitung eingesetzt. Als Arzneidroge werden üblicherweise die Wurzel und das Rhizom verwendet (Rhodiolae radix, Rhodiolae rhizoma). "Rosavin" bezeichnet den Stoff Rosavin (s. Formel unten). Wird der Begriff "Rosavine" verwendet, so bezieht er sich entweder auf den Einzelstoff Rosavin, oder er umfasst die Stoffe Rosavin, Rosin und Rosarin.

Für die Wirkungen sind in erster Linie Glykoside, darunter Salidrosid (Rhodiolosid) und Rosavin, sowie das Aglykon p-Tyrosol (4-(2-Hydroxyethyl)phenol), verantwortlich. Daneben enthält die Pflanze zahlreiche weitere Inhaltsstoffe, zum Beispiel Flavonoide, Proanthocyanidine, organische Säuren und Terpenoide. Maßgebliche Leitsubtanzen sind: 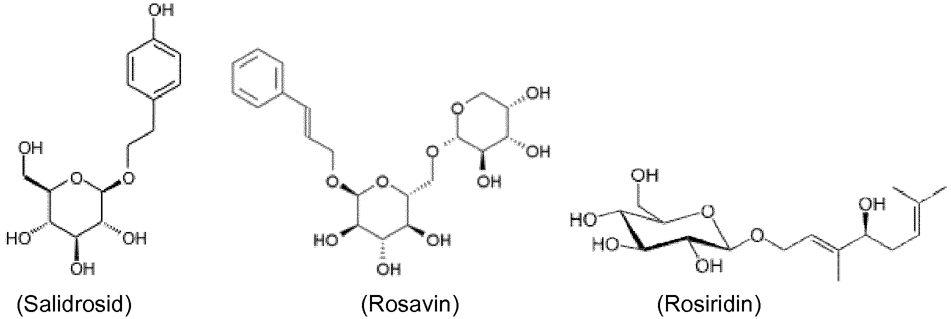 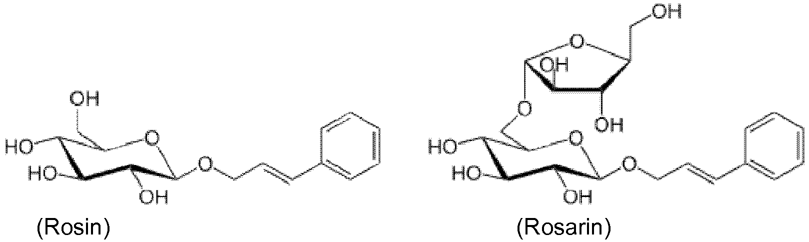 Der IUPAC Name von Rosavin ist (2E)-3-phenylprop-2-en-1-yl 6-O-α-L-arabinopyranosyl-α-D-glucopyranosid, eine alternative Bezeichnung ist (2S,3R,4S,5S,6R)-2-[(E)-3-phenylprop-2-enoxy]-6-([(2S,3R,4S,5S)-3,4,5-trihydroxyoxan-2-yl]oxymethyl)oxane-3,4,5-triol. Die CAS Nr. von Rosavin ist 84954-92-7. Der IUPAC Name von Salidrosid ist 2-(4-hydroxyphenyl)ethyl β-D-glucopyranosid, alternative Bezeichnungen sind Rhodiolosid, (2R,3S,4S,5R,6R)-2-(hydroxymethyl)-6-[2-(4-hydroxyphenyl)ethoxy]oxane-3,4,5-triol, oder Tyrosol Glucosid. Die CAS Nr. von Salidrosid ist 10338-51-9. Weitere Bestandteile von der Rosavin enthaltenden Komponente können Rosin (Cinnamyl-O-β-D-glucopyranosid; IUPAC Name (2R,3S,4S,5R,6R)-2-(hydroxymethyl)-6-[(E)-3-phenylprop-2-enoxy]oxane-3,4,5-triol; CAS Nr. 85026-55-7) und Rosarin (Cinnamyl-(6'-O-α-Larabinopyranosyl)-O-β-D-glucopyranosid; IUPAC Name (2E)-3-Phenyl-2-propen-1-yl-6-O-α-L-arabinofuranosyl-β-D-glucopyranosid; CAS Nr. 84954-93-8) sein. Die Rosarine enthaltende Komponente, in Form von Rosenwurzpulver oder Rosenwurzextrakt kann die folgenden Wirkstoffe enthalten: Phenylpropanderivate (Rosavin, Rosin und Rosarin (so genannten Rosavine), Phenylethylderivate (Salidrosid (Rhodiolosid), p-Tyrosol), Flavonoide (Rodiolin, Rodionin, Rodiosin, Acetylrodalgin, Tricin), Monoterpene (Rosiridol, Rosaridin), Triterpene (Daucosterol, β-Sitosterol) und Phenolsäuren (Chlorogen-, Hydroxyzimt- und Gallussäuren); Leitsubstanzen sind Rosavin und Salidrosid.

Eine aufgrund des zusätzlichen Gehalts an Flavonoiden bevorzugte Rosavine enthaltende Komponente ist Rosenwurzpulver. Das Pulver wird vorzugsweise aus der ganzen Pflanze gewonnen (*Rhodiola rosea* L.), kann jedoch auch aus der Wurzel gewonnen werden (rhizoma et radix). Alternativ kann auch Rosenwurzextrakt eingesetzt werden. Hierbei werden die Wirkstoffe von Rosenwurz aus dem Wurzelstock oder der gesamten Pflanze durch Extraktion gewonnen. Die Pulver/Extrakte weisen bevorzugt einen Gehalt von ca. 3 - 6% Rosavinen und ca. 0,8 - 1,2% Salidrosid auf. Ein ebenfalls verwendbarer Extrakt ist *Rhodiola rosea* SHR-5 Extrakt (DER 2.5-5:1, erstes Extraktsionsmittel Ethanol 70%, zweites Extraktsionsmittel Wasser); dieser Extrakt enthält etwa 2.5%-2.7% Rhodioloside (= Salidroside), etwa 3.9%-6% Rosavin und etwa 0.8% Tyrosol. Rosenwurzpulver aus der ganzen Pflanze mit einem Gehalt an Rosavin von ca. 4% und einem Gehalt von Salidrosid von ca. 0,9-1,1% ist besonders bevorzugt.

*Rhodiola rosea* Pulver oder Extrakt enthält erfindungsgemäß einen Rosavin-Gehalt von wenigstens ca. 1%, 2%, 3%, 4%, 5%, 6%, 7%, 8% vorzugsweise ca. 2-6% oder ca. 3- 5%, besonders bevorzugt ca. 4%. Der Salidrosid Gehalt beträgt ca. 0,2% bis ca. 4%, vorzugsweise ca. 0,5% bis ca. 2%, besonders bevorzugt ca. 0,8% bis ca. 1,5% und insbesondere etwa 0,9% bis 1,1%; alle Angaben in Gew-%.

### 4.3 Die Linalool und Linalylacetat enthaltende Komponente (iii):

Der hierin verwendete Begriff "Linalool und Linalylacetat enthaltende Komponente" kann eine Mischung aus den reinen Stoffen Linalool und Linalylacetat bezeichnen, eine Kombination dieser Mischung mit einem oder weiteren Stoffen, einen Pflanzenextrakt, der diese Mischung enthält, eine Kombination aus Pflanzenextrakten, die diese Mischung enthalten, die Kombination aus einem Pflanzenextrakt, der diese Mischung enthält, mit einem oder weiteren Stoffen, sowie die Kombination aus einer Kombination aus Pflanzenextrakten, die diese Mischung enthalten, mit einem oder weiteren Stoffen. Diese Stoffe können dem Fachmann bekannte Trägerstoffe oder Hilfsstoffe für die Herrichtung einer pharmazeutischen Zusammensetzung sein, insbesondere die erfindungsgemäßen mittelkettigen Triglyceride (MCT) und andere Öle, Wachse und Fette.

In einer bevorzugten Ausführungsform der erfindungsgemäßen Komponente (iii) enthält oder ist diese Komponente ein Pflanzenextrakt. In einer besonders bevorzugten Ausführungsform enthält oder ist diese Komponente ein Pflanzenextrakt aus den Blüten des *Lavandula officinalis.* Ebenso kann erfindungsgemäß jeder andere Pflanzenextrakt verwendet werden, der einen erfindungsgemäßen Anteil an Linalool und Linalylacetat besitzt (z.B. Muskatellersalbei *Salvia sclarea,* Bergamott *Citrus bergamia*). In einer besonders bevorzugten Ausführungsform ist der Pflanzenextrakt der Komponente (iii) der Lavendelblütenextrakt der Firma Flavex mit der Typ Nummer 084.001.

Die erfindungsgemäße Komponente (iii) umfasst Pflanzenextrakt aus Blüten des *Lavandula officinalis* aus CO₂-Extraktionsverfahren (Hochdruckextraktion). Ausgeschlossen von der vorliegenden Erfindung sind Lavendelextrakte, die durch wässrige Extraktion, Wasserdampfextraktion oder Extraktion mit Alkohol gewonnen wurden. Diese wässrigen und alkoholischen Extrakte sind keine wirksamen Komponenten im Sinne der vorliegenden Erfindung.

Erfindungsgemäße CO₂-Extrakte aus Lavendelblüten enthalten hohe Anteile an Linalool und Linalylacetat, ferner u. a. Ocimen, 1.8 Cineol, Lavandulol, Terpinen-4-ol, Lavandulylacetat, Caryophyllen, Sesquilavandulol.

### Linalool besitzt zwei enantiomere Formen:

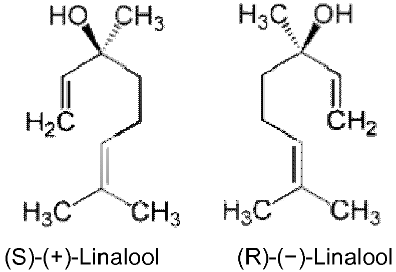

Alternative Bezeichnungen von Linalool sind Linalylalkohol bzw. Licareol ((R)-Form) und Coriandrol ((S)-Form). Die CAS Nummern für Linalool sind 78-70-6 (Racemat), 126-91-06 [(R)-(-)-Linalool] und 126-90-9 [(S)-(+)-Linalool]. Linalool ist Bestandteil vieler ätherischer Öle. Es kommt im Koriander, Hopfen, Muskat, Ingwer, Bohnenkraut, Zimt, Basilikum, Majoran, Thymian, Oregano, schwarzen Pfeffer, Safran und anderen Gewürzpflanzen vor.

Linalylacetat findet sich als Hauptkomponente im Lavendelöl (30 - 60 %), im Lavandinöl (25 - 50 %), im Muskatellersalbeiöl (bis zu 75 %) und im Bergamottenöl (30 - 45 %) und kommt darüber hinaus in vielen anderen ätherischen Ölen vor. Es besitzt zwei enantiomere Formen:

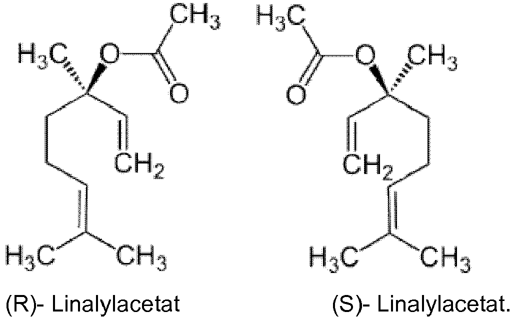

Die CAS Nummern für Linalylacetat sind 115-95-7 [(RS)-Linalylacetat], 16509-46-9 [(R)-Linalylacetat] und 51685-40-6 [(S)-Linalylacetat]. Weitere Bezeichnungen für Linalylacetat sind Essigsäurelinalylester, Ethansäurelinalyester, (3,7-Dimethylocta-1,6-dien-3-yl) acetat und Bergamiol.

Die Linalool enthaltende Komponente (iii) kann Ocimene, 1,8-Cineol, Lavandulol, Terpinen-4-ol, Carophyllene und Sesquilavandulole enthalten.

*Ocimene:* die folgenden Substanzen sind Ocimene, die in Lavendelblütenextrakt enthalten sein können:

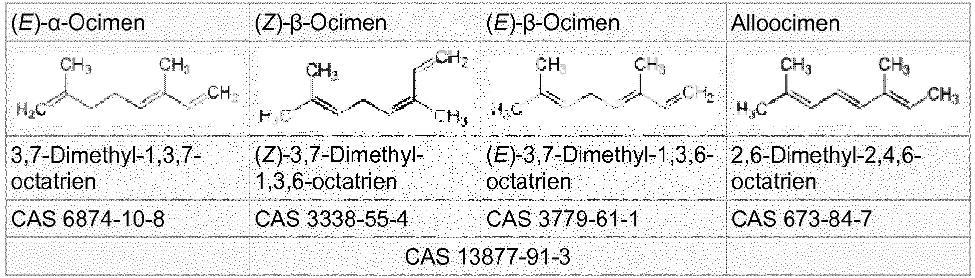

*1,8 Cineol:* 1,8-Cineol kommt in größeren Mengen in Eukalyptus (Eukalyptusöl enthält ungefähr 85 % Cineol) und Lorbeer vor, aber auch in Lavendel, Minze, Heilsalbei, Thymian, Basilikum und im Teebaum. Synonyme Bezeichnungen sind Eucalyptol, Limonen-1,8-oxid, 1,8-Epoxy-p-menthan, oder 1,3,3-Trimethyl-2-oxabicyclo[2.2.2]octan.

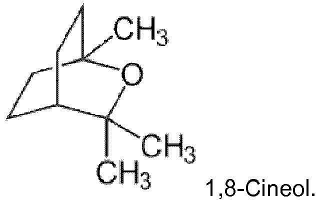

*Lavandulol* kommt hauptsächlich in Lavendel vor und ist der Trivialname der azyklischen Verbindung (±)-2-Isopropenyl-5-methyl-4-hexen-1-ol. Es kommt in zwei Stereoisomeren vor (CAS Nummer 58461-27-1)

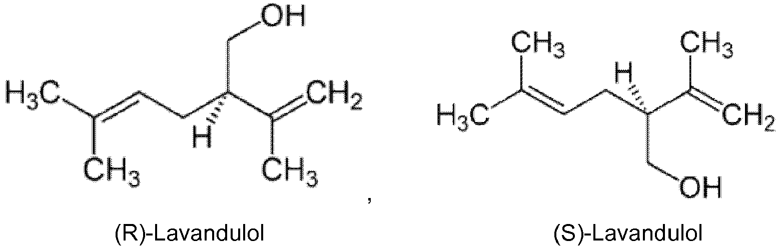

*Terpinen-4-ol* ist Bestandteil vieler ätherischer Öle, zum Beispiel der Pflanzengattungen Pinus und Eucalyptus, oder des Lavendelöls oder Teebaumöls. Synonyme Bezeichnungen sind 1-p-Menthen-4-ol und 4-Carvomenthenol. Die CAS Nummer ist 562-74-3 (Racemat). Es ist in Wasser praktisch unlöslich. *Lavandulylacetat* ist die Acetatform des Lavanulols:

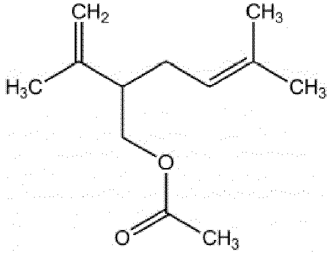

Synonyme Bezeichnungen sind 2-isopropenyl-5-methylhex-4-en-1-yl acetate; 4-hexen-1-ol, 5-methyl-2-(1-methylethenyl)-acetate; Acetic acid 2-isopropenyl-5-methyl-hex-4-enylester, die CAS-Nr. ist 25905-14-0.

*Caryophyllene* sind Sesquiterpene, die in ätherischen Ölen, z. B. in Lavendel, Basilikum, Rosmarin, Zimt, Oregano, Kümmel und Pfeffer enthalten sind. Es existieren α-Caryophyllen (Humulen) und β-Caryophyllen (E-BCP, für (E)-BetaCaryophyllen)).

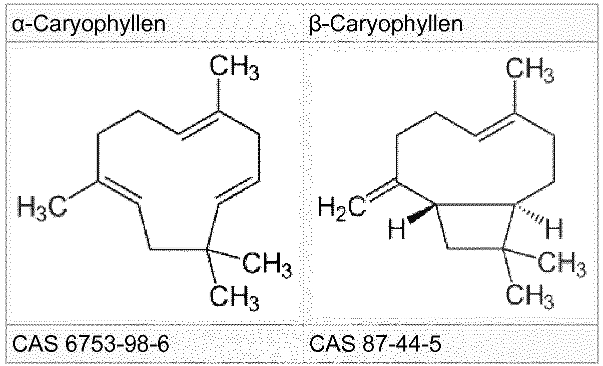

*Sesquilavandulole* sind wasserunlösliche Substanzen und kommen in verschiedenen Formen vor. 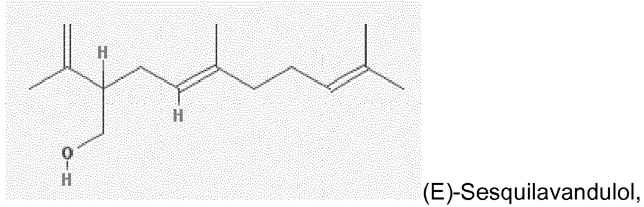 oder (4E)-5,9-dimethyl-2-prop-1-en-2-yldeca-4,8-dien-1-ol), CAS Nr. 120707-27-9 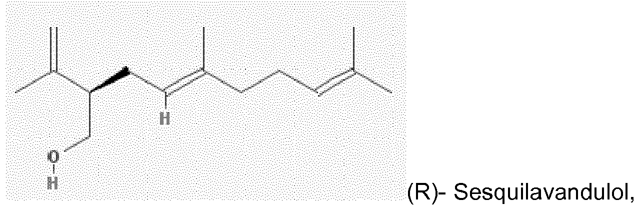 oder (2R,4E)-5,9-dimethyl-2-prop-1-en-2-yldeca-4,8-dien-1-ol 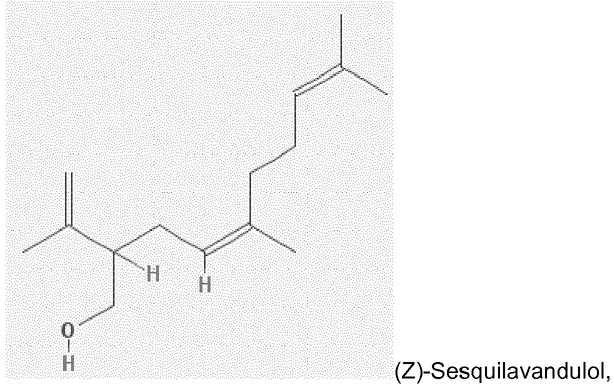 oder (4Z)-5,9-dimethyl-2-prop-1-en-2-yldeca-4,8-dien-1-ol)

Die Linalool enthaltende Komponente (iii) enthält bevorzugterweise wenigstens 40 % (w/w) Linalool/Linalylacetat und besonders bevorzugt wenigstens 50 % (w/w), wenigstens 55 % (w/w) oder wenigstens 60 % (w/w) Linalool/Linalylacetat. In einer bevorzugten Ausführungsform der vorliegenden Erfindung umfasst die Linalool enthaltende Komponente der pharmazeutische Zusammensetzung 15 bis 95 % (w/w) Linalool/Linalylacetat, bevorzugt 40%-90% (w/w). Besonders bevorzugt sind hierbei 55-85% oder circa 70 % (w/w) Linalool/Linalylacetat. Hierbei kann in einer spezifischen Ausführungsform einer Linalool enthaltenden Komponente (iii) der Anteil von Linalool bevorzugt größer als etwa 25% und der Anteil von Linalylacetat etwa 30 bis etwa 45% (w/w) sein.

Bevorzugterweise beträgt der Anteil an Myrcen, Ocimen, p-Ocimen, Limonen, Lavandulol, Terpinen-4-ol, alpha-Terpineol, Lavandulylacetat und Caryophyllen der Komponente (iii) weniger als jeweils 2,5% (w/w), und der Anteil an 1,8 Cineol beträgt vorzugsweise weniger als 15% (w/w). Vorzugsweise ist der Anteil von Myrcen, Ocimen, p-Ocimen, Limonen, Lavandulol, Terpinen-4-ol, alpha-Terpineol, Lavandulylacetat, Caryophyllen und 1,8 Cineol zusammengenommen weniger als etwa 15% (w/w).

Die Bestandteile des Auszugs aus Blüten des Arzneilavendels haben nicht nur eine anxiolytische Wirkung, sondern zeigen auch antidepressive Effekte. Eine Metaanalyse von zwei placebokontrollierten Studien bestätigte einen signifikanten Effekt auf depressive Verstimmung. An den Untersuchungen hatten Patienten mit subsyndromaler Angsterkrankung teilgenommen. Angehörige der Placebogruppen hatten initial bei der "depressiven Stimmung", dem Item 6 der Hamilton-Angst-Skala (HAMA), im Mittel einen Wert von 2,1 Punkten aufgewiesen. Nach zehnwöchiger Therapie mit Lavendelblütenextrakt sank der Mittelwert auf 0,9 Punkte. Angehörige der Placebogruppen erreichten dagegen noch einen Mittelwert von 1,2 - ein statistisch signifikanter Unterschied.

### 4.4 Die S-Adenosylmethionin enthaltende Komponente (iv):

Der hierin verwendete Begriff "S-Adenosylmethionin enthaltende Komponente" kann den reinen Stoff S-Adenosylmethionin oder eine Kombination des reinen Stoffs S-Adenosylmethionin mit einem oder weiteren Stoffen bezeichnen. Diese Stoffe können dem Fachmann bekannte Trägerstoffe oder Hilfsstoffe für die Herrichtung einer pharmazeutischen Zusammensetzung sein, insbesondere die erfindungsgemäßen mittelkettigen Triglyceride (MCT) und andere Öle, Wachse und Fette. S-Adenosylmethionin (SAM, oder SAMe) ist ein als Reinsubstanz erhältlicher Stoff, der sowohl intravenös als auch zur oralen Verabreichung geeignet ist. S-Adenosylmethionin ist ein Aminosäurederivat, das natürlich in allen Zellen des Körpers vorkommt. SAMe spielt bei verschiedenen Funktionen des Gehirns eine wichtige Rolle und fördert die Synthese von Adrenalin aus Noradrenalin und die Bildung von Dopamin und verbessert die Funktion von Neurotransmittern. Neben der Synthese von Neurotransmittern ist SAMe auch in den Metabolismus von Hormonen (wie den Katecholaminen durch die COMT) involviert.

In einer Ausführungsform der vorliegenden Erfindung umfasst die pharmazeutische Zusammensetzung 5 bis 500 mg S-Adenosylmethionin pro Einzeldosis, bevorzugt 10 mg bis 200 mg pro Dosis, besonders bevorzugt 25 bis 75, oder 30 bis 65 mg oder etwa 50 mg pro Dosis. Besonders bevorzugt sind hierbei 50 mg S-Adenosylmethionin pro Einzeldosis. Die erfindungsgemäße Zusammensetzung enthält pro Einzeldosis (450-650 mg) vorzugsweise etwa 50 mg SAMe.

### 4.5 Die Linolsäure und Linolensäure enthaltende Komponente (v):

Der hierin verwendete Begriff "Linolsäure und Linolensäure enthaltende Komponente" kann eine Mischung aus den reinen Stoffen Linolsäure und Linolensäure bezeichnen, eine Kombination dieser Mischung mit einem oder weiteren Stoffen, einen Pflanzenextrakt der diese Mischung enthält, eine Kombination aus Pflanzenextrakten, die diese Mischung enthalten, die Kombination aus einem Pflanzenextrakt, der diese Mischung enthält, mit einem oder weiteren Stoffen, sowie die Kombination aus einer Kombination aus Pflanzenextrakten, die diese Mischung enthalten, mit einem oder weiteren Stoffen. Diese Stoffe können dem Fachmann bekannte Trägerstoffe oder Hilfsstoffe für die Herrichtung einer pharmazeutischen Zusammensetzung sein, insbesondere die erfindungsgemäßen mittelkettigen Triglyceride (MCT) und andere Öle, Wachse und Fette.

In einer bevorzugten Ausführungsform der erfindungsgemäßen pharmazeutischen Zusammensetzung enthält oder ist die Komponente (v) ein Pflanzenextrakt. In einer besonders bevorzugten Ausführungsform ist die Komponente (v) ein Pflanzenextrakt aus Samen des *Salvia hispanica.*

Der erfindungsgemäße Pflanzenextrakt aus Samen des *Salvia hispanica* (auch Chiasamen-Öl genannt) hat ein hohen Gehalt an mehrfach ungesättigten Fettsäuren, bestehend aus 15-20% Linolsäure (C18:2) und> 60% alpha Linolensäure (C18:3). INC-Name (CTFA): Salvia Hispanica (Chia) Seed Extract, CAS-Nr. 93384-40-8, EINECS-Nr. 297-250-8. Es kann ferner Rosmarinus Officinalis (Rosemary) Leaf Extract, CAS-Nr. 84604-14-8, EINECS-Nr. 283-291-9 (INCI Key G: nicht mehr als 0,1 %) enthalten. In einer besonders bevorzugten Ausführungsform ist der Pflanzenextrakt der Komponente (v) der Chia Samen-Extrakt der Firma Flavex mit der Typ Nummer 165.002.

*Linolsäure* oder (cis,cis)-Octadeca-9,12-diensäure ist eine zweifach ungesättigte Fettsäure mit 18 Kohlenstoffatomen (18:2). Sie gehört aufgrund der Lage ihrer zweiten Doppelbindung zur Gruppe der Omega-6-Fettsäuren. Linolsäure kommt als Ester chemisch gebunden in vielen Triglyceriden vor, die Hauptanteil der natürlichen fetten Öle sind. Neben Chiasamen-Öl weisen auch andere natürliche Öle einen hohen Gehalt an Linolsäure auf, z. B. Traubenkernöl (58-78 %), Distelöl (Safloröl) (55-81%), Hanföl (etwa 50 %), Sojaöl (49-57 %), Baumwollsaatöl (45-58 %), Weizenkeimöl (40-55 %), Maiskeimöl (34-62 %), Sonnenblumenöl (20-75 %) und Rapsöl (18-30 %).

*α-Linolensäure* (alpha-Linolensäure, oft auch nur Linolensäure oder ALA), oder (all-cis)-Octadeca-9,12,15-triensäure, ist eine dreifach ungesättigte Fettsäure mit 18 Kohlenstoffatomen und gehört zur Gruppe der Omega-3-Fettsäuren. α-Linolensäure findet sich als Ester chemisch gebunden in vielen Triglyceriden, welche den Hauptanteil der natürlichen Fette und Öle ausmachen. Neben Chiasamen-Öl weisen auch andere natürliche Öle einen hohen Gehalt an α-Linolensäure auf, z. B. das Öl aus dem Iberischen Drachenkopf (bis zu 70%), Perillaöl (31-42%), Leinöl (56-71 %), Hanföl (28 %), Walnussöl (ca. 15 %), Rapsöl (5-16 %) und Sojaöl (4-11 %). Manche pflanzlichen Öle enthalten nur geringe Mengen (bis 1,5 %) an α-Linolensäure, dazu zählen Sonnenblumenöl, Olivenöl und Traubenkernöl (weniger als 1 % α-Linolensäure, jedoch 72 % Linolsäure).

Vorzugsweise erfolgt die Herstellung des Chiasamen-Öls durch Hochdruckextraktion mit natürlicher Quellkohlensäure (CO₂-Extraktion), die Extrakte weisen daher keine Lösungsmittelrückstände und keine Rückstände von anorganischen Salzen und Schwermetallen sowie keine vermehrungsfähigen Keime auf. Bevorzugterweise wird der in der pharmazeutischen Zusammensetzung verwendete Chiasamen-Extrakt mit 290 ppm Carnosol und Carnosolsäure aus Rosmarin als Antioxidans stabilisiert.

Alternativ können als Komponente (v) auch einzelne Omega-3 Fettsäuren verwendet werden, bzw. Mischungen verschiedener Omega-3 Fettsäuren. Insbesondere können Mischungen aus Omega-3 und Omega-6 Fettsäuren verwendet werden.

Die Linolsäure/Linolensäure enthaltende Komponente (v) enthält bevorzugterweise wenigstens 40 % (w/w) Linolsäure/Linolensäure und besonders bevorzugt wenigstens 60 % (w/w), wenigstens 70 % (w/w) oder wenigstens 75 % (w/w) Linolsäure/Linolensäure. In einer bevorzugten Ausführungsform der vorliegenden Erfindung umfasst die Linolsäure/Linolensäure enthaltende Komponente der pharmazeutischen Zusammensetzung 40-95 % (w/w) Linolsäure/Linolensäure, bevorzugt 50-90% (w/w). Besonders bevorzugt sind hierbei 65-85% oder circa 80 % (w/w) Linolsäure/Linolensäure. Hierbei kann in einer spezifischen Ausführungsform einer Linolsäure/Linolensäure enthaltenden Komponente (v) der Anteil von Linolsäure bevorzugt größer als etwa 10% (w/w) und kleiner als etwa 40% (w/w), bevorzugt 15-25% (w/w) sein und der Anteil von Linolensäure größer als etwa 50% (w/w), bevorzugt größer als etwa 60% (w/w) sein.

Ferner kann die Linolsäure/Linolensäure enthaltende Komponente (v) Palmitoleinsäure (z.B. C16:1 w7), Stearinsäure (z.B. C18:0), Vaccensäure (z.B. C18:3 w3), Arachinsäure (z.B. C20:0), Palmitinsäure (z.B. C16:0) und Ölsäure (z.B. C18:1 w7) enthalten. Bevorzugterweise beträgt der Anteil an anderen Fettsäuren ausgewählt aus Palmitoleinsäure (z.B. C16:1 w7), Stearinsäure (z.B. C18:0), Vaccensäure (z.B. C18:3 w3) und Arachinsäure (z.B. C20:0) der Komponente (v) weniger als jeweils 2,5% (w/w), und der Anteil an Palmitinsäure (z.B. C16:0) und Ölsäure (z.B. C18:1 w7) beträgt weniger als 15% (w/w), vorzugsweise weniger als10% (w/w). Vorzugsweise ist der Anteil von Palmitoleinsäure (z.B. C16:1 w7), Stearinsäure (z.B. C18:0), Vaccensäure (z.B. C18:3 w3), Arachinsäure (z.B. C20:0), Palmitinsäure (z.B. C16:0) und Ölsäure (z.B. C18:1 w7) zusammengenommen weniger als etwa 20% (w/w).

Omega-3-Fettsäuren haben einen wichtigen Anteil an einer anti-depressiven Therapie. Es wird angenommen, dass ein Mangel an Omega-3-Fettsäuren die Verbindung zwischen den psychischen Symptomen und den häufig begleitend auftretenden körperlichen Symptomen herstellt.

### 4.6 Komponente (vi) enthaltend einen Aminosäurekomplex und optional Mikronährstoffe, sowie weitere optionale Komponenten

Bei Aminosäuremangel können, besonders in neurologischen Bereichen, massive Störungen auftreten. Darum kann ein Aminosäurekomplex zu den anderen obengenannten Inhaltsstoffen hinzugefügt werden. Ein für die erfindungsgemäße Anwendung verwendbarer Aminosäurekomplex umfasst eine Kombination von L-Tryptophan, Glutamin, L-Tyrosin HCl, Acetyl-L-Carnitin, Phenylalanin. In einer besonderen Ausführungsform besteht der Aminosöurekomplex aus einer Kombination von L-Tryptophan, Glutamin, L-Tyrosin HCl, Acetyl-L-Carnitin und Phenylalanin. Ein erfindungsgemäßer Aminosäurekomplex als Teil der Komponente (iv) oder als Komponente (iv) als solches umfasst eins, zwei, drei, vier, fünf oder mehr Elemente aus der Gruppe bestehend aus L-Glutaminsäure, L-Lysin, L-Prolin, L-Leucin, L-Aspartat, L-Valin, L-Isoleucin, L-Serin, L-Threonin, L-Arginin, L-Alanin, L-Methionin, L-Histidin, L-Phenylalanin, L-Tyrosin, L-Glutamin, L-Tryptophan, L-Cystein, L-Selenocystein und L-Carnitin und jegliche beliebige Kombination hiervon in einer Konzentration, die ausreichend ist, um wenigstens 5% oder wenigstens 10%, vorzugsweise wenigstens 15% der Tagesdosis eines Erwachsenen zu decken. Besonders bevorzugt enthält der Aminosäurekomplex wenigstens ein, zwei, drei, vier oder mehr bzw. alle essentiellen Aminosäuren ausgewählt aus der Gruppe bestehend aus Phenylalanin, Leucin, Methionin, Lysin, Isoleucin, Valin, Threonin, Tryptophan, Histidin, und Cystein sowie jegliche Kombinationen hiervon.

Ferner kann die oben genannte Zusammensetzung optional Mikronährstoffe enthalten. Zu den Mikronährstoffen zählen Vitamine (Vitamine A, B, C, D, E und K), Mineralstoffe (wie Kalzium, Kalium oder Magnesium), Spurenelemente (z. B. Eisen, Zink, Selen und Mangan). Erfindungsgemäße Mikronährstoffe oder molekulare Mikronährstoffe als Teil der Komponente (vi) umfassen eins, zwei, drei, vier, fünf oder mehr Elemente aus der Gruppe bestehend aus Vitamin A, B₁, B₂, B₃ (Niacin), B₅ (Pantothensäure), B₇ (Biotin), B₉ (Folsäure), B₁₂, C, D, E, K, Cobalt, Eisen, Fluor, Jod, Kupfer, Mangan, Molybdän, Selen, Silicium, Vanadium, Zink, Zinn, Arsen, Bor und Rubidium in jeder beliebigen Kombination in einer Konzentration, die ausreichend ist, um wenigstens 5% oder wenigstens 10%, vorzugsweise wenigstens 15% der Tagesdosis eines Erwachsenen zu decken. Besonders bevorzugte Mikronährstoffe sind Vitamin B-Komplex (Vitamine B1, B2, B6, B12, Biotin, Niacin und Pantothensäure), Folsäure und L-Carnitin. Die Mikronährstoffe können in Form eines Gemüseextrakts vorliegen.

Weiterhin kann die erfindungsgemäße Zusammensetzung eine L-Dopa enthaltende Komponente umfassen, vorzugsweise ist diese Komponente ein Extrakt aus *Mucuna Pruriens.* Bei manchen Depressionen können auch unausgeglichene Neurotransmitter ein Rollen spielen. In diesem Zusammenhang wird meistens von Serotonin und Noradrenalin gesprochen, aber auch ein Mangel an Dopamin kann eine Rolle spielen. *Mucuna pruriens* (L-Dopa) kann im Zusammenhang mit der Behandlung von Depressionen in Kombination mit der o.g. Kombination eingesetzt werden, da Sie stressbedingte Ursachen, welche auf Dopamin zurückzuführen sind, lindern kann. Der wirksame Bestandteil von *Mucuna pruriens* (Juckbohne) ist L-Dopa (3,4-Dihydroxyphenylalanin). Es können Dosierungen von 5, 10, 20, 30, 40, 50, und 100 mg/kg Mucuna pruriens Extrakt eingesetzt werden (mit einem L-Dopa Gehalt von ca. 15% bis ca. 40%). Bevorzugte Extrakte sind Samenextrakte aus *Mucunia pruriens* L..

### 4.7 Trägerstoff: Mittelkettige Triglyceride (MCT)

Die oben genannten Komponenten (i) bis (vi) werden vorzugsweise in MCT Öle eingearbeitet. Erfindungsgemäße MCT-Öle werden auch als Mittelkettige Triglyceride (MKT), Medium-chain-Triglyceride (MCT) oder MCT-Fette bezeichnet und sind Triglyceride, die mittelkettige Fettsäuren enthalten. Mittelkettige Fettsäuren sind die Capron- (C 6:0), Capryl- (C 8:0), Caprin- (C 10:0) und die Laurinsäure (C 12:0). Es handelt sich dabei um gesättigte Fettsäuren, welche vor allem in tropischen Pflanzenfetten wie Kokosfett (ca. 60 %) und Palmkernöl (ca. 55 %) vorkommen. Zu einem geringen Teil sind sie auch im Milchfett (ca. 10 %) enthalten. Vorzugsweise wird 100% MCT-Öl mit mittelkettigen Triglyceride aus Palm- und Kokosfett verwendet. Im nachfolgenden wird der Begriff MCT-Öl verwendet, der Begriff bezieht sich auf Einzelsubstanzen (Capronsäure), aber ebenso auf Mischungen von verschiedenen MCT-Ölen (z.B. Mischung aus einem, zwei oder mehreren von Capron-, Capryl-, Caprin- und Laurinsäure). In einer Ausführungsform umfassen die MCT Öle vorwiegend Caprylsäure 50-80% (w/w) und Caprinsäure (20-50% w/w), während Capronsäure, Laurinsäure und Myristinsäure weniger als jeweils 4% (w/w) der MCT Öle ausmachen und insgesamt zusammengenommen weniger als 10% des MCT-Öls ausmachen.

### 4.8. Hilfsstoffe, Zusatzstoffe

Ein bevorzugter Trägerstoff ist MCT-Öl bzw. eine Mischung aus MCT-Ölen. Weitere mögliche Trägerstoffe sind Stärke, Maltodextrin und Schwefelverbindungen. Weitere mögliche Zusatzstoffe sind Antioxidantien wie Tocopherole, Carotinoide, Natriumsulfit, Natriummetabisulfit, Ascorbinsäure und andere. Ferner kann die pharmazeutische Zusammensetzung weitere Hilfsstoffe enthalten, wie Füllmittel, Bindemittel, Sprengmittel (Zerfallsmittel) und Gleitmittel. Vorzugsweise ist die pharmazeutische Zusammensetzung jedoch frei von Hilfsstoffen und anderen Zusatzstoffen.

### 4.9. Herstellung der pharmazeutischen Zusammensetzung

Die Herstellung der erfindungsgemäßen Zusammensetzung erfolgt durch Mischen der verschiedenen Komponenten. Vorzugsweise werden die Komponenten in einem pharmazeutisch verträglichen Trägerstoff gemischt. Insbesondere bevorzugt ist der Trägerstoff MCT-Öl oder MCT-Fett. MCT-Fette sind aufgrund ihrer kürzeren Fettsäurenkettenlänge im wässrigen Milieu relativ gut löslich. MCT-Öl wird daher schnell und direkt in der Blutbahn aufgenommen und zur Leber transportiert. Die Verdauung und Resorption von MCT-Öl vollzieht sich unabhängig von Helfersubstanzen, wie etwa Gallensäuren. Die Aufnahme von Proteinen bzw. Aminosäuren und verschiedenen Mineralien, wie Magnesium und Calcium, wird durch MCT-Öl verbessert.

Als nicht-limitierendes Beispiel zur Herstellung von Kapseln mit den oben genannten Komponenten wird das MCT-Öl leicht auf eine Temperatur von etwa 34 bis 38 °C erwärmt. Nacheinander werden die einzelnen Komponenten in das MCT-Öl eingearbeitet; zunächst wird SAM im MCT-Öl aufgelöst, anschließend wird die Hyperforin enthaltende Komponente (z.B. reines Hyperforin) erwärmt und hinzugegeben. Dann werden Mikronährstoffe (wie Vitamin B-Komplex, L-Carnitin) mit Omega-3 Ölen und der Kava Kava und/oder Rosavin enthaltenden Komponente leicht erwärmt und ebenfalls eingearbeitet. Als nächster Schritt wird Lavendelöl Extrakt hinzugegeben und das entstandene Produkt mittels eines Stabes nochmals mit einer Umdrehungszahl von mindestens etwa 30.000 U/min gut vermischt. Nach einer Wartezeit von ca. zwei Stunden wird es abschließend in Kapseln abgefüllt.

Alternativ können alle Komponenten einzeln in MCT-Öl aufgelöst oder eingebracht werden und die verschiedenen Mischungen werden dann abschließend vereint.

### 4.10. Konfektionierung

Eine Einzeldosis der pharmazeutischen Zusammensetzung enthält üblicherweise insgesamt 20 mg bis 5000 mg an aktiven Substanzen der Komponenten (i) bis (v), vorzugsweise insgesamt 50 mg bis 1000 mg, oder 100 mg bis 500 mg, besonders bevorzugt enthält eine Dosis der pharmazeutischen Zusammensetzung insgesamt 125 mg bis 300 mg an aktiven Substanzen der Komponenten (i) bis (v). In einer speziellen Ausführungsform enthält eine Dosis der pharmazeutischen Zusammensetzung insgesamt etwa 150-200 mg an aktiven Substanzen der Komponenten (i) bis (v).

Eine Einzeldosis der pharmazeutischen Zusammensetzung enthält die Hyperforin enthaltende Komponente (i) in einer Menge, die ausreichend ist, um eine Menge von 1-1000 mg, bevorzugt 5-500mg, 10-250mg, besonders bevorzugt 20-100mg oder 30-75mg (Gesamt-) Hyperforin pro Dosis zu ergeben. In einer speziellen Ausführungsform enthält eine Dosis der pharmazeutischen Zusammensetzung die Hyperforin enthaltende Komponente (i) in einer Menge, die ausreichend ist, um eine Menge von etwa 40-60mg oder speziell etwa 50 mg Hyperforin, oder Gesamt-Hyperforin pro Dosis zu ergeben.

Eine Einzeldosis der pharmazeutischen Zusammensetzung enthält die Kavapyrone enthaltende Komponente (ii) in einer Menge, die ausreichend ist, um eine Menge von 1-1000 mg, bevorzugt 5-300mg, 10-200mg, besonders bevorzugt 15-100mg oder 20-70mg (Gesamt-) Kavapyrone pro Dosis zu ergeben. In einer speziellen Ausführungsform enthält eine Dosis der pharmazeutischen Zusammensetzung die Kavapyrone enthaltende Komponente (ii) in einer Menge, die ausreichend ist, um eine Menge von etwa 25-45mg oder speziell etwa 35 mg Kavapyrone pro Dosis zu ergeben. Eine Einzeldosis der pharmazeutischen Zusammensetzung enthält die Rosavine enthaltende Komponente (ii) in einer Menge, die ausreichend ist, um Rosavine in einer Menge von ca. 0,1 bis ca. 20 mg, bevorzugt, 0,4 bis 8 mg, besonders bevorzugt 0,6 bis 4mg und insbesondere bevorzugt etwa 1-2 mg Rosavine pro Dosis zu ergeben. In einer speziellen Ausführungsform enthält die Komponente etwa 1,6 mg an Rosavinen (und etwa 0,4 mg an Salidrosid). Die Dosierung der Komponente (z.B. Rosenwurzpulver) kann entsprechend dem Rosavin Gehalt Komponente eingestellt werden.

Einzeldosen für *Rhodiola rosea* Pulver/Extrakte sind beispielsweise wie folgt: Für Pulver/Extrakte mit 1% Rosavin ergeben sich Dosierungen von 40 mg bis 800 mg, für Pulver/Extrakte mit 2% Rosavin Dosierungen von 20 mg bis 400 mg, für Pulver/Extrakte mit 3% Rosavin Dosierungen von 15 mg bis 300 mg, für Pulver/Extrakte mit 4% Rosavin Dosierungen von 10 mg bis 200 mg und für Pulver/Extrakte mit 5% Rosavin Dosierungen von etwa 8 mg bis 125 mg. Die angegebenen Mengen beziehen Gewichtsprozent.

Andere Dosierungen bei abweichenden Rosavingehalten, sowie Zwischenwerte zwischen den o.g. Werten können vom Fachmann anhand dieser Angaben ermittelt werden und sind von der Erfindung mit umfasst.

Eine Einzeldosis der pharmazeutischen Zusammensetzung enthält die Linalool/Linalylacetat enthaltende Komponente (iii) in einer Menge, die ausreichend ist, um eine Menge von 0,5-1000 mg, bevorzugt 1-300mg, 2-150mg, besonders bevorzugt 5-100mg oder 10-50mg Linalool/Linalylacetat pro Dosis zu ergeben. In einer speziellen Ausführungsform enthält eine Dosis der pharmazeutischen Zusammensetzung die Linalool/Linalylacetat enthaltende Komponente (iii) in einer Menge, die ausreichend ist, um eine Menge von etwa 15-35mg oder speziell etwa 25 mg Linalool/Linalylacetat pro Dosis zu ergeben. Das Verhältnis von Linalool zu Linalylacetat beträgt hierbei zwischen 1:10 und 10:1, bevorzugt zwischen 2:1 und 1:2 und besonders bevorzugt etwa 1:1.

Eine Einzeldosis der pharmazeutischen Zusammensetzung enthält die S-Adenosylmethionin enthaltende Komponente (iv) in einer Menge, die ausreichend ist, um eine Menge von 0,5-1000 mg, bevorzugt 1-300mg, 2-150mg, besonders bevorzugt 5-100mg oder 10-50mg S-Adenosylmethionin pro Dosis zu ergeben. In einer speziellen Ausführungsform enthält eine Dosis der pharmazeutischen Zusammensetzung die S-Adenosylmethionin enthaltende Komponente (iv) in einer Menge, die ausreichend ist, um eine Menge von etwa 15-35mg oder speziell etwa 25 mg S-Adenosylmethionin pro Dosis zu ergeben.

Eine Einzeldosis der pharmazeutischen Zusammensetzung enthält die Linolsäure/Linolensäure enthaltende Komponente (v) in einer Menge, die ausreichend ist, um eine Menge von 1-1000 mg, bevorzugt 5-300mg, 10-200mg, besonders bevorzugt 15-100mg oder 20-70mg Linolsäure und/oder Linolensäure pro Dosis zu ergeben. In einer speziellen Ausführungsform enthält eine Dosis der pharmazeutischen Zusammensetzung die Linolsäure/Linolensäure enthaltende Komponente (v) in einer Menge, die ausreichend ist, um eine Menge von etwa 25-45mg oder speziell etwa 35 mg Linolsäure und/oder Linolensäure pro Dosis zu ergeben.

Eine Einzeldosis der pharmazeutischen Zusammensetzung enthält üblicherweise 5 mg bis 2000 mg Aminosäurekomplex oder Aminosäurekomplex und Mikronährstoffe, vorzugsweise 15-1000 mg oder 30-500 mg, besonders bevorzugt 50-250 mg oder 75-200 mg. In einer spezifischen Ausführungsform enthält eine Dosis der pharmazeutischen Zusammensetzung etwa 150 mg an Aminosäurekomplex oder Aminosäurekomplex und Mikronährstoffen.

Um eine Einzeldosis der pharmazeutischen Zusammensetzung herzustellen, ist eine Menge MCT-Öl notwendig, die etwa der ein- bis fünffachen, vorzugsweise der etwa zwei- bis vierfachen, besonders bevorzugt etwa der dreifachen Menge an Hyperforin entspricht (w/w). Alternativ macht das MCT-Öl etwa die Hälfte bis etwa einem Viertel der Summe der anderen Komponenten aus, bevorzugt etwa ein Drittel.

Die oben genannten Komponenten, bzw. deren Wirkstoffe können in jedem beliebigen Verhältnis zueinander (Komponente (i) : (ii) : (iii) : (iv) : (v) : Aminosäurekomplex/Mikronährstoffe : MCT-Öl) gemischt werden, solange die erforderlichen Mindestmengen bzw. Höchstmengen enthalten sind.

Entsprechend den obigen Angaben umfasst eine Einzeldosis eine Gesamtmenge von etwa 100 bis 5000 mg, vorzugsweise etwa 150-2000 mg, etwa 200-1500 mg, besonders bevorzugt etwa 300-1000 mg oder 350-750 mg der vorgenannten Bestandteile. Vorzugsweise wird eine Einzeldosis der erfindungsgemäßen Zusammensetzung in Kapseln zu 450 bis 650 mg konfektioniert. In einer speziellen Ausführungsform ist die Einzeldosis als Kapsel zu etwa 500 mg konfektioniert. Als nicht-limitierendes Beispiel umfasst eine Einzeldosis:
(i) etwa 50 mg Hyperforin
(ii) etwa 35 mg Kavapyrone
(iii) etwa 25 mg Linalool/Linalylacetat
(iv) etwa 25 mg SAMe
(v) etwa 35 mg alpha Linolensäure
etwa 150 mg Aminosäurekomplex/Mikronährstoffe
etwa 150 mg MCT-Öl.
Als weiteres Beispiel umfasst eine Einzeldosis
(i) etwa 50 mg Hyperforin
(ii) etwa 1,6 mg Rosavin
(iii) etwa 25 mg Linalool/Linalylacetat
(iv) etwa 25 mg SAMe
(v) etwa 35 mg alpha Linolensäure
etwa 150 mg Aminosäurekomplex/Mikronährstoffe
etwa 150 mg MCT-Öl.

Werden beispielsweise Pflanzenextrakte für die Herstellung einer Einzeldosis der pharmazeutischen Zusammensetzung verwendet enthält eine Einzeldosis der erfindungsgemäßen Zusammensetzung beispielsweise die folgenden Mengen der einzelnen Komponenten:
(i) ca. 120 mg Johanniskrautextrakt mit einem Gehalt von ca. 50 mg Hyperforin
(ii) ca. 50 mg Kava Kava - Extrakt mit einem Gehalt von ca. 35 mg an Kavapyronen, und/oder ca. 40 mg Rosenwurzpulver (*Rhodiola rosea* L., gesamte Pflanze) mit einem Gehalt von ca. 1,6 mg Rosavin und ca. 0,36-0,44 mg an Salidrosid
(iii) ca. 40 mg Lavendelextrakt mit einem Gehalt von ca. 12 mg Linalool und ca. 13 mg Linalylacetat
(iv) ca. 25 mg S-Adenosylmethionin (SAMe)
(v) ca. 50 mg Chia-Samenextrakt mit einem Gehalt von ca. 35 mg alpha-Linolensäure, sowie optional
(vi) ca. 150 mg Aminosäurekomplex und molekulare Mikronährstoffe, [50 mg L-Trp, 20 mg L-Glu, 20 mg L-Thyrosin-HCI; 30 mg L-Acetyl- Carnitin, 20mg DL-Phenylalanin; Mikronährstoffe =5 mg Vitamin B komplex und 5 mg Gemüseextrakt]
   (i) bis (vi) sind hierbei vermischt mit ca. 150 mg MCT-Öl (als Trägerstoff).

Der Begriff "Einzeldosis" bezieht sich auf eine entsprechend konfektionierte Menge der pharmazeutischen Zusammensetzung, also z.B. auf eine Tablette oder eine Kapsel (im o.g. Beispiel entspricht eine Einzeldosis einer Kapsel mit ca. 420-440 mg an Komponenten (i) bis (vi)) und mit MCT Öl von ca. 560-600 mg.

### 4.11. Darreichungsformen

Die erfindungsgemäße Zusammensetzung kann in jeglicher Form zubereitet werden, in welcher Medikamente üblicherweise vorliegen z.B. in Form einer Tablette, Kapsel, Pille, Pastille oder eines Pulvers, einer essbaren Zusammensetzung, eines Nahrungsergänzungsmittels oder eines Getränks. Alternative Darreichungsformen sind Suspension, auch weiterverarbeitete Nahrungsmittel (wie z.B. Snackriegel oder als Beimengung in Schokolade oder als Trinkpulver). Als bevorzugte Darreichungsform ist die Zusammensetzung in Kapselform aufbereitet.

### 4.12. Dosierung

Die Dosierung richtet sich einerseits nach dem Schweregrad der depressiven Erkrankung und andererseits nach den pharmakokinetischen Eckdaten des zu behandelnden Patienten (Körpergewicht, evtl. verminderte oder verstärkte Resorptionsfähigkeit) und kann vom Fachmann entsprechend den beobachteten Grundvoraussetzungen angepasst werden.

Die Dosierung der pharmazeutischen Zusammensetzung ist dergestalt, dass die folgenden Tagesdosen der einzelnen Komponenten erreicht werden:
(i) die Hyperforin enthaltende Komponente in einer Menge, dass der Patient eine Tagesdosis pro kg Körpergewicht von 0,1-20 mg, vorzugsweise 0,25-15 mg, besonders bevorzugt 0,5-10 mg oder etwa 1-5 mg Hyperforin oder Gesamthyperforin erhält;
(ii) die Kavapyrone enthaltende Komponente in einer Menge, dass der Patient eine Tagesdosis pro kg Körpergewicht von 0,1-15 mg, vorzugsweise 0,2-10 mg, besonders bevorzugt 0,35-5 mg oder etwa 0,7-3,5 mg (Gesamt-) Kavapyrone erhält; bzw. die Rosavine enthaltende Komponente in einer Menge, dass der Patient eine Tagesdosis pro kg Körpergewicht von ca. 5 µg bis ca. 1 mg, bevorzugt 20 µg bis 0,4 mg, besonders bevorzugt 30 µg bis 0,25 mg und insbesondere bevorzugt etwa 50 µg bis 100 µg Rosavine pro kg Körpergewicht erhält.
(iii) die Linalool/Linalylacetat enthaltende Komponente in einer Menge, dass der Patient eine Tagesdosis pro kg Körpergewicht von 0,1-15 mg, vorzugsweise 0,2-10 mg, oder 0,3-7,5 mg besonders bevorzugt 0,4-5 oder etwa 0,5-2,5 mg Linalool/Linalylacetat erhält;
(iv) die SAMe enthaltende Komponente in einer Menge, dass der Patient eine Tagesdosis pro kg Körpergewicht von 0,1-15 mg, vorzugsweise 0,2-10 mg, oder 0,3-7,5 mg besonders bevorzugt 0,4-5 oder etwa 0,5-2,5 mg SAMe erhält; und
(v) die Linolsäure/Linolensäure enthaltende Komponente in einer Menge, dass der Patient eine Tagesdosis pro kg Körpergewicht von 0,1-15 mg, vorzugsweise 0,2-10 mg, besonders bevorzugt 0,35-5 mg oder etwa 0,7-3,5 mg Linolsäure/Linolensäure erhält,
wobei die Dosierung einer jeweiligen Komponente unabhängig von der Dosierung der anderen Komponenten ist.

In einer spezifischen Ausführungsform umfasst die pharmazeutische Zusammensetzung in einer Einzeldosis:
(i) etwa 50 mg Hyperforin
(ii) etwa 35 mg Kavapyrone und/oder etwa 1,6 mg Rosavin
(iii) etwa 25 mg Linalool/Linalylacetat
(iv) etwa 25 mg SAMe
(v) etwa 35 mg alpha Linolensäure.

Die Behandlung mit der erfindungsgemäßen Zusammensetzung erfolgt vorzugsweise in mehreren Phasen, in welchen die Dosierung nach und nach reduziert wird. Vorzugsweise umfasst das Dosierungsschema wenigstens drei Phasen, eine Anfangsphase und zwei darauf folgende Phasen, in welchen die Dosierung zurückgefahren wird.

Die Dosierung während der Anfangsphase richtet sich nach dem Körpergewicht der zu behandelnden Person in einer Ausführungsform der Erfindung vorzugsweise wie folgt:
(a) Bis 60kg Körpergewicht: Anfangsdosierung: Je nach Schweregrad 3 bis 4 vier Kapseln (eine Kapsel entspricht einer Einzeldosis) täglich; für die Dauer von 7-14 Tagen, vorzugsweise etwa 10 Tagen, hiervon eine morgens-, eine bis zwei mittags und eine abends. Vorzugsweise erfolgt die Einnahme nach dem Essen.

Anschließend wird die Dosierung für die folgenden 7-14 Tage, vorzugsweise 10 Tage (Tag 11 - 20) lang dann auf 2 Kapseln täglich reduziert (jeweils 1 morgens und abends nach dem Essen).

Anschließend (vorzugsweise ab 21. Tag) wird die Dosierung - je nach Bedarf - beibehalten oder auf 1 Kapsel täglich reduziert. Bei einer Einnahme von nur 1 Kapsel tgl. wird diese vorzugsweise morgens nach dem Essen eingenommen.
(b) Über 60 kg Körpergewicht: Anfangsdosierung: Für die ersten 7-14 Tagen, vorzugsweise etwa 10 Tage je nach Schweregrad 4 - 5 Kapseln täglich; eine bis zwei morgens, zwei mittags und eine abends. Vorzugsweise erfolgt die Einnahme nach dem Essen.

Die anschließenden Folgephasen (vorzugsweise ab 11. Tag) folgen dem oben beschriebenen Schema für Patienten mit einem Körpergewicht bis 60 kg.

Die oben unter (a) und (b) beschriebenen Dosierungen stellen empfohlene Maximaldosierungen dar. Bei größeren Körpergewichten über 100 kg kann die Dosierung jedoch auf maximal 6 Kapseln täglich erhöht werden.

Die oben angegebenen Dosierungen können jederzeit während der Behandlung auch unterschritten werden oder auch - ohne Nebenwirkungen - beendet werden, wenn eine Besserung eintritt. Üblicherweise tritt eine signifikante Verbesserung der Krankheitszustände innerhalb von 2-5 Wochen ein.

### 4.13. Bevorzugte Verfahren zur Gewinnung der wirksamen Bestandteile

Bevorzugt werden die wirksamen Bestandteile mittels Hochdruckextraktion mit Kohlendioxid als Lösungsmittel gewonnen. Überkritisches Kohlendioxid ist ein allgemein als sicher anerkanntes Lösungsmittel für lipophile Inhaltsstoffe. Das Hochdruckverfahren (bis 500 bar) arbeitet ohne Temperaturbelastung unter Ausschluss von Sauerstoff und bewahrt auf diese Weise die Wirksamkeit der wertvollen Pflanzeninhaltsstoffe. CO₂-Extrakte haben ein natürlicheres Aroma, einen frischeren Duft und eine bessere Wirksamkeit. Bei den bevorzugten Extraktionsprozessen mit Kohlendioxid als Lösungsmittel wird das Kohlendioxid für die Extraktion in den überkritischen Zustandsbereich gebracht. Der Vorteil dieses Verfahrens ist in den guten Lösungsmitteleigenschaften begründet, die Kohlendioxid in diesem Zustandsbereich aufweist. Im Zustandsbereich für die Extraktion besitzt das Kohlendioxid Viskositäten im Bereich 30-150 µPas. Aufgrund dieser niedrigen Viskositäten können Stoffübergangsprozesse leicht stattfinden und die wirksamen Bestandteile in einer hohen Konzentration und Reinheit aus dem Basismaterial gewonnen werden. Die kritische Dichte des Kohlendioxids beträgt 468 kg·m⁻³. Im überkritischen Zustand kann die Dichte des Kohlendioxids gut über Temperatur und Druck bis hin zu flüssigkeitsähnlichen Dichten variiert werden. Die Löslichkeit bzw. Selektivität für bestimmte Substanzen ist stark abhängig von der Dichte und kann über diesen Parameter daher gut beeinflusst und gesteuert werden.

Die Naturstoffextrakte können gebrauchsfertig kommerziell bezogen werden, z.B. von FLAVEX Naturextrakte GmbH, Nordstraße 7, D-66780 Rehlingen. Einzelstoffe der erfindungsgemäßen Zusammensetzung können auch von Feinchemikalien-Herstellern bezogen werden, wie z.B. von Denk Feinchemie GmbH, Prinzregentenstr. 79, D-81675 München).

### 4.14 Anwendung in der Behandlung von Depression

Eine Depression ist eine psychische Störung. Die folgenden spezifischen Krankheitsbilder werden von der internationalen Klassifizierung (ICD 10) als Depression erfasst und können mit der erfindungsgemäßen Zusammensetzung behandelt werden.

Depressive Episode (F32.-): Bei den typischen leichten (F32.0), mittelgradigen (F32.1) oder schweren (F32.2 und F32.3) Episoden leidet der betroffene Patient unter einer gedrückten Stimmung und einer Verminderung von Antrieb und Aktivität. Die Fähigkeit zu Freude, das Interesse und die Konzentration sind vermindert. Ausgeprägte Müdigkeit kann nach jeder kleinsten Anstrengung auftreten. Der Schlaf ist meist gestört, der Appetit vermindert, Selbstwertgefühl und Selbstvertrauen sind fast immer beeinträchtigt. Sogar bei der leichten Form kommen Schuldgefühle oder Gedanken über eigene Wertlosigkeit vor. Die gedrückte Stimmung verändert sich von Tag zu Tag wenig, reagiert nicht auf Lebensumstände und kann von so genannten "somatischen" Symptomen begleitet werden, wie Interessenverlust oder Verlust der Freude, Früherwachen, Morgentief, deutliche psychomotorische Hemmung, Agitiertheit, Appetitverlust, Gewichtsverlust und Libidoverlust. Abhängig von Anzahl und Schwere der Symptome ist eine depressive Episode als leicht, mittelgradig oder schwer zu bezeichnen. Bei schweren Depressionen können auch Suizidgedanken und Suizidhandlungen hinzu kommen. Depression schließt einzelne Episoden von depressiver Reaktion, psychogener Depression und reaktiver Depression (F32.0, F32.1, F32.2) ein.

Rezidivierende depressive Störung (F33): Hierbei handelt es sich um eine Störung, die durch wiederholte depressive Episoden (F32.-) charakterisiert ist. In der Anamnese finden sich dabei keine unabhängigen Episoden mit gehobener Stimmung und vermehrtem Antrieb (Manie). Kurze Episoden von leicht gehobener Stimmung und Überaktivität (Hypomanie) können allerdings unmittelbar nach einer depressiven Episode, manchmal durch eine antidepressive Behandlung mitbedingt, aufgetreten sein. Die schwereren Formen der rezidivierenden depressiven Störung (F33.2 und -.3) haben viel mit der manisch-depressiven Krankheit, der Melancholie, der vitalen Depression und der endogenen Depression gemeinsam. Die erste Episode kann in jedem Alter zwischen Kindheit und Senium auftreten, der Beginn kann akut oder schleichend sein, die Dauer reicht von wenigen Wochen bis zu vielen Monaten. Rezidivierende depressive Störung schließen rezidivierende Episoden (F33.0 oder F33.1) wie depressive Reaktion, psychogene Depression, reaktive Depression und Saisonale depressive Störungen ein.

Anhaltende affektive Störungen (F34): Hierbei handelt es sich um anhaltende und meist fluktuierende Stimmungsstörungen, bei denen die Mehrzahl der einzelnen Episoden nicht ausreichend schwer genug sind, um als leichte depressive Episoden gelten zu können. Da sie jahrelang, manchmal den größeren Teil des Erwachsenenlebens, andauern, ziehen sie beträchtliches subjektives Leiden und Beeinträchtigungen nach sich. Zu den anhaltenden affektiven Störungen gehören Zyklothymia (F34.0), eine andauernde Instabilität der Stimmung mit zahlreichen Perioden von Depression und leicht gehobener Stimmung (Hypomanie), von denen aber keine ausreichend schwer und anhaltend genug ist, um die Kriterien für eine bipolare affektive Störung (F31.-) oder rezidivierende depressive Störung (F33.-) zu erfüllen, affektive Persönlichkeit(-sstörung), zykloide Persönlichkeit und zyklothyme Persönlichkeit, sowie Dysthymia (F34.1), eine chronische, wenigstens mehrere Jahre andauernde depressive Verstimmung, die weder schwer noch hinsichtlich einzelner Episoden anhaltend genug ist, um die Kriterien einer schweren, mittelgradigen oder leichten rezidivierenden depressiven Störung (F33.-) zu erfüllen. Dysthemia schließt anhaltende ängstliche Depressionen wie Neurose, depressive Persönlichkeit(-sstörung) sowie neurotische Depression ein.

Bipolare Störungen werden wie folgt eingeteilt: Bipolar I (ICD 10: F30 und F31): mindestens eine manische Episode; Bipolar II (ICD 10: F31.8): rezidivierende Depressionen mit Hypomanien (Hypomanie: ohne ausgeprägte Beeinflussung von Arbeit oder sozialer Aktivität, gehobene Stimmung über mindestens 4 Tage); Zyklothymia (ICD 10: F34.0): chronische Phasen leichter Depressionen und Hypomanien, weniger als 2 Monate pro Jahr symptomfrei; gemischte/dysphorische Manie (ICD 10: F31.6): Bipolar I-Störung mit gleichzeitigem Vorliegen manischer und depressiver Symptome. Weiterhin werden die folgenden Verlaufsformen der bipolaren Erkrankungen unterschieden: Rapid cycling (ICD 10: F31.8): mindestens 4 Phasen pro Jahr (in ca. 20 % der Fälle); ultra rapid cycling (ICD 10: F31.8): Phasenwechsel innerhalb von Wochen oder Tagen, mehr als 4 Episoden pro Monat sowie ultradian cycling (ICD 10: F31.8): Phasenwechsel innerhalb eines Tages, an mehr als 4 Tagen in der Woche.

Der diagnostische und statistische Leitfaden psychischer Störungen (DSM-IV-TR), bzw. deren 2013 revidierte Fassung, DSM-5, ist ein Ersatz und/oder eine Ergänzung für die jeweiligen Passagen im ICD-10. Entsprechend den obigen Ausführungen werden die folgenden Zustände als Depression im Sinne der vorliegenden Erfindung erachtet:

| **ICD-10** | | **DSM-5** | |
|---|---|---|---|
| **F30** | **Manische Episode** | | |
| F30.0 | Hypomanie | | |
| F30.1 | Manie ohne psychotische Symptome | | |
| F30.2 | Manie mit psychotischen Symptomen | | |
| F30.8 | Sonstige manische Episode | | |
| F30.9 | Manische Episode, n.n.b. | | |
| | | | |
| | **Bipolare affektive Störung** | | **Bipolar-I-Störung** |
| **F31** | gegenwärtig: | | aktuelle oder letzte Episode: |
| F31.0 | hypomane Episode | 296.40 | hypoman |
| | | | *oder* Bipolar-II-Störung (296.89) |
| | | | |
| F31.1 | manische Episode ohne psychotische | | manisch |
| | Symptome | 296.41 | leicht |
| | | 296.42 | mittelschwer |
| | | 296.43 | schwer ohne psychot.Merkmale |
| F31.2 | manische Episode mit psychotischen | 296.44 | mit psychotischen Symptomen |
| | Symptomen | 296.45 | in teilweiser Remission |
| | | 296.46 | vollremittiert |
| | | 296.40 | nicht näher bezeichnet |
| | | | |
| | | | depressiv |
| F31.3 | leichte oder mittelgr. depr. Episode | 296.51 | leicht |
| | | 296.52 | mittelschwer |
| F31.4 | schwere depr. Episode ohne psychot. | 296.53 | schwer ohne psychot. Merkmale |
| | Sympt. | 296.54 | mit psychotischen Merkmalen |
| F31.5 | schwere depressive Episode mit | 296.55 | in teilweiser Remission |
| | psychot. Sympt. | 296.56 | vollremittiert |
| | | 296.50 | nicht näher bezeichnet |
| | | | |
| F31.6 | gemischte Episode | 296.7 | Bipolar-I-Störung, letzte Episode nicht näher bez. |
| F31.7 | remittiert | | |
| | | | |
| F31.8 | Sonstige bipolare affektive Störung | 296.89 | Bipolar-II-Störung *oder* |
| | | 296.89 | Andere näher bezeichnete bipolare u. verwandte Störung |
| F31.9 | Bipolare affektive Störung, nnb | 296.80 | Nicht näher bezeichnete Bipolare Störung |
| | | | |
| **F32** | **Depressive Episode** | **296.2x** | **Major Depression, einzelne Episode** |
| F32.1 | Leichte depressive Episode | 296.21 | leicht |
| F32.2 | Mittelgradige depressive Episode | 296.22 | mittelschwer |
| F32.3 | Schwere depr. E. ohne psychot. Sym. | 296.23 | schwer ohne psychot. Merkmale |
| F32.3 | Schwere depr. E. mit psychot. Sym. | 296.24 | mit psychotischen Merkmalen |
| | | 296.25 | in teilweiser Remission |
| | | 296.26 | vollremittiert |
| F32.8 | Sonstige depressive Episoden | 296.20 | nicht näher bezeichnet |
| F32.9 | Depressive Episode nnb | 311 | Andere näher bezeichnete oder nicht näher bezeichnete depressive Störung |
| | | | |
| **F33** | **Rezidivierende depressive Störung** | **296.3x** | **Major Depression, rezidivierend** |
| | gegenwärtig: | | |
| F33.0 | leichte Episode | 296.31 | leicht |
| F33.1 | mittelgradige Episode | 296.32 | mittelschwer |
| F33.2 | schwere E. ohne psychot. Symptome | 296.33 | schwer ohne psychot. Merkmale |
| F33.3 | schwere E. mit psychot. Symptomen | 296.34 | mit psychotischen Merkmalen |
| | | 296.35 | in teilweiser Remission |
| F33.4 | remittiert | 296.35 | vollremittiert |
| F33.8 | Sonstige rezidiv. depressive Störung | 296.30 | nicht näher bezeichnet |
| F33.9 | Rez. depressive Störung, nnb | 311 | Andere näher bezeichnete oder nicht näher bezeichnet depressive Störung |
| | | | |
| **F34** | **Anhaltende affektive Störung** | | |
| F34.0 | Zyklothymia | 301.13 | Zyklothyme Störung |
| F34.1 | Dysthymia | 300.4 | Anhaltende depressive Störung (Dysthymia) |
| F34.8 | Sonstige anhaltende affekt. Störung | | |
| F34.9 | Anhaltende affekt. Störung, nnb | | |
| | | | |
| **F38** | **Andere affektive Störungen** | 311 | Andere näher bezeichnete oder nicht näher bezeichnet depressive Störung |
| F38.0 | Andere einzelne affektive Störungen | | |
| F38.1 | Andere rezidiv. affekt. Störungen | | |
| F38.8 | Sonstige näher bez. affekt. Störung | | |
| | | | |
| **F39** | **Nicht näher bez. affekt. Störungen** | 311 | Andere näher bezeichnete oder nicht näher bezeichnet depressive Störung |
| | **Weitere Kategorien:** | | |
| | | | |
| F25.0 | Schizoaffektive Störung, bipolar | 295.70 | Schizoaffektive Störung |
| F25.0 | Schizoaffektive Störung, depressiv | | |
| | | 296.99 | Disruptive Mood Dysregulation Disorder |
| | | 625.4 | Premenstrual Dysphoric Disorder |
| | | | |
| F06.3 | Organische affektive Störungen | 293.83 | Bipolare und verwandte Störung aufgrund eines medizinischen Krankheitsfaktors |
| F1x.8 | Psychische und Verhaltensstörungen durchpsychotrope Substanzen: | | |
| | Sonstige u. Verhaltensstörungen | | Substanzinduzierte affektive Störungen: |
| | | 291.89 | Alkohol |
| | | 292.84 | Amphetamine, Kokain, Opiate etc. |

Weiterhin unter den Begriff Depression im Sinne der vorliegenden Erfindung zählen die folgenden, nicht mehr in der ICD-10 reflektierten Störungen: postpartale Depression, Schwangerschaftsdepression, somatisierte Depression, agitierte Depression, atypische Depression (AD), sowie Spät- oder Involutionsdepression.

Zur Diagnose einer Depression kann ein Fachmann den Patientenfragebogen PHQ-9 verwenden (Deutsche Übersetzung und Validierung des "Brief Patient Health Questionnaire (Brief PHQ)" durch B. Löwe, S. Zipfel und W. Herzog, Medizinische Universitätsklinik Heidelberg, Englische Originalversion: Spitzer, Kroenke & Williams, 1999) (siehe Annex 1). Der Fragebogen PHQ-9 umfasst neun Fragen zur Depressivität. Er wurde als Screening-Instrument zur Diagnostik von Depressivität für den routinemäßigen Einsatz im somatisch-medizinischen Bereich entwickelt. Anders als viele andere Fragebogen zur Depressivität erfasst der PHQ-9 mit jeder Frage eines der neun DSM-Kriterien für die Diagnose der "Major Depression". Der PHQ-9 wird von der DSM-5-Arbeitsgruppe der American Psychiatric Association als Instrument zur Messung des Schweregrades der Major Depression nach den neuen DSM-5-Kriterien, sowie auch von der kassenärztlichen Vereinigung Niedersachsen und den fachärztlichen und psychotherapeutischen Berufsverbänden empfohlen.

*Auswertung des PHQ-9:* Der PHQ-9 kann sowohl dimensional wie auch kategorial ausgewertet werden. Die dimensionale Auswertung dient der Bestimmung Schweregrade der Depressivität und kann somit insbesondere zur Verlaufsdiagnostik sowie zur Beurteilung des Therapieeffektes herangezogen werden. Die kategoriale Auswertung dient der Diagnosestellung einer Major Depression (Diagnose Major Depression ja vs. nein) und orientiert sich am Vorgehen zur Diagnosestellung einer Major Depression. Um die Diagnose der Major Depression müssen insgesamt 5 der 9 abgefragten Symptome als mindestens "an mehr als der Hälfte der Tage" gegeben eingestuft werden. Davon muss ein Symptom entweder Interesselosigkeit (Item a.) oder Niedergeschlagenheit (item b.) sein. Item i gilt bereits dann als erfüllt, wenn es mit "an einzelnen Tagen" beantwortet wird.

**Kategorialer Auswertungsalgorithmus für den PHQ-9**

| **Syndrom** | **Frage** | **Algorithmus** |
|---|---|---|
| Major Depressives Syndrom | a.-i. | Fünf oder mehr der Fragen a. - i. sind mit mindestens "an mehr als der Hälfte der Tage" beantwortet; unter diesen befindet sich auch Frage a. oder b. (i. wird auch dann mitgezählt, wenn es mit "an einzelnen Tagen" beantwortet ist) |
| Andere Depressive Syndrome | a.-i. | Zwei, drei oder vier der Fragen a. - i. sind mit mindestens "an mehr als der Hälfte der Tage" beantwortet; unter diesen befindet sich auch Frage a. oder b. (i. wird auch dann mitgezählt, wenn es mit "an einzelnen Tagen" beantwortet ist) |

Dimensionale Auswertung (Schweregrad der Depression): Der Skalensummenwert "Depressivität" kann unter Verwendung der neun Items des PHQ-9 berechnet werden. Dazu werden den Antwortkategorien folgende Werte zugewiesen:
0 ("Überhaupt nicht"),
1 ("An einzelnen Tagen"),
2 ("An mehr als die Hälfte der Tage") und
3 ("Beinahe jeden Tag")
Der Skalensummenwert "Depressivität" (hier erfindungsgemäß auch als Gesamtscore, oder Score bezeichnet) entspricht somit der Summe der Punktwerte und liegt zwischen 0 und 27. Folgende Interpretation der möglichen Skalensummenwerte wird herangezogen (nach Kroenke et al. J Gen Intern Med. 2001 Sep; 16(9): 606-613)):

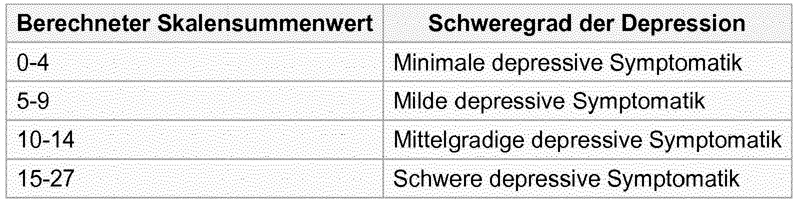

Werte <5 beschreiben eine nicht pathologische, minimale depressive Symptomatik.

*Testdiagnostische Gütekriterien und Kriteriumsvalidität von PHQ-9:* Eine Meta-Analyse mit insgesamt 14 klinischen Studien (insgesamt 5026 Patienten, davon 770 mit Major Depressive Syndrom) zeigte eine hohe Sensitivität von 80 % wie auch Spezifizität von 92 % über die Studien auf. Bei kategorialer Auswertung weist der PHQ-9 eine Sensitivität von 78 % (psychosomatische Patienten) bzw. 86 % (medizinische Patienten) und eine entsprechende Spezifität von 80 % bzw. 94 % für die Diagnose der Major Depression auf. Für die Diagnose aller depressiven Störungen weist der PHQ-9 eine Sensitivität von 78 % (psychosomatische Patienten) bzw. 75 % (medizinische Patienten) und eine Spezifität von 71 % bzw. 90 % auf. Die interne Konsistenz des PHQ-9 erwies sich als sehr gut mit Cronbachs alpha = 0.88 bzw. 0.89. Auch die Test-Retest-Reliabilität ist sehr gut. Der PHQ-9 ist außerdem als Telefoninterview validiert worden und weist hier gute Werte auf. Der PHQ-9 weist eine gute Änderungssensitivität bei Verwendung des Summenwertes auf, so dass er auch in Längsschnittuntersuchungen, z.B. zur Messung von Therapieeffekten, verwendet werden kann. Damit stellt der PHQ-9 ein verlässliches Diagnoseinstrument zu Bewertung von depressiven Erkrankungen und Depressionen dar.

Die vorliegende Erfindung bezieht sich auf die Verwendung einer pharmazeutischen Zusammensetzung wie hierin definiert zur Behandlung einer Depression in einem Patienten. Die Erfindung bezieht sich ferner auf ein Heilverfahren zur Behandlung einer Depression, umfassend die Verabreichung einer pharmazeutischen Zusammensetzung wie hierin definiert an einen unter Depression leidenden Patienten. Vorzugsweise ist der Patient ein Mensch.

Ferner kann die erfindungsgemäße Zusammensetzung zur Behandlung von Schlafstörungen (Ein- und Durchschlafstörungen) eingesetzt werden. Die Schlafstörung kann eine Insomnie, eine Dyssomnie oder eine Parasomnie sein. Insomnien bezeichnen Schwierigkeiten beim Einschlafen, Störungen des Durchschlafens und vorzeitiges Erwachen. Als Dyssomnien (intrinsische Dyssomnie) werden alle primären Erkrankungen des Schlafens und/oder Wachens, bei denen die Erholungsfunktion des Schlafes gestört ist, bezeichnet. Als Parasomnien bezeichnet man Störungen, die aufgrund körperlicher und psychischer Faktoren in den Schlafprozess einbrechen.

### 4.15 Sonstige Definitionen

Der Begriff "etwa" und der Begriff "circa" (ca.) bezeichnen eine dem Fachmann geläufige geringe Abweichung des auf den jeweiligen Begriff folgenden numerischen Werts. Zum Zwecke der vorliegenden Anmeldung bedeuten die Begriffe "etwa" und "circa" (ca.) eine Abweichung von 10%, bevorzugt 5%, weiter bevorzugt 1% des darauf folgenden Werts, d.h. die Angabe "ca. 50%" umfasst einen Wert von 45% bis 55%, oder bevorzugt von 47,5% bis 52,5% oder weiter bevorzugt 49,5% bis 50,5%, die Angabe "etwa 500 mg" bezieht sich auf 450-550 mg oder bevorzugt 475-525 mg, weiter bevorzugt auf 495 mg bis 505 mg.

### 5. Beispiele

### 5.0 Vorangegangene Studien

In vorangegangenen Studien wurden alle Stoffe auch einzeln verabreicht, jedoch nur mit Teilerfolgen. Gegen schwere und mittelschwere Depressionen konnte keine Heilwirkung erreicht werden. Ebenso wurde beispielsweise eine Kombination aus Hyperforin, KavaKava und Chia-Konzentrat in DHA Öl untersucht. Auch andere Kombinationspräparate aus Baldiran Johanniskruat und Passionsfrucht, ggf. auch mit Hopfen zeigten bei mittelgradigen und schweren Depressionen keine zufriedenstellende Wirkung. Mit diesen Kombinationen konnten nur bei leichten Stimmungsschwankungen und Angstzuständen Teilerfolge erzielt werden. Bei schweren Depressionen (bipolaren Störungen) konnten jedoch keine Erfolge erzielt werden.

Weitere Versuche mit ansatzweise vielversprechenden Kombinationen haben letztlich zu der erfindungsgemäße Zusammensetzung geführt, wie sie in den folgenden Beispielen exemplarisch dargelegt ist und in den Ansprüchen beschrieben ist.

Bei der vorliegenden Erfindung wurden die in den vorangegangenen Versuchen gewonnenen Erkenntnisse genutzt, inwiefern die Pflanzenstoffe unterschiedlich absorbiert werden und nach der Resorption einer teilweisen Metabolisierung unterliegen, die erst dazu führt, dass die gesamten zugeführten Stoffe in aktiver Form im Blut zur Verfügung stehen, und dass die verschiedenen Wirkstoffe durch die Magen-Darm-Flora des Patienten auch unterschiedlich resorbiert und abgebaut werden.

### 5.1.1 Herstellung von Kapseln enthaltend die pharmazeutische Zusammensetzung

Das Herstellungsverfahren beruht auf die Einarbeitung der verschiedenen Komponenten in MCT Öl/MCT-Ölen.

In das MCT-Trägeröl werden mit dem Stabmixer bei einer Temperatur von 34 - 38 Grad Celsius zunächst das Hyperforin, anschließend die Kavapyrone, die Lavendelextrakte, das SA-Me, der Chia-Samenextrakt und abschließend der Aminosäurekomplex/Mikronährstoffe eingebracht. Nach ca. einer zweistündigen Wartezeit wird die Mischung nochmals mit einer Umdrehungszahl von mindestens 30.000/Minute vermischt und abschließend mit einem Mindestgewicht von ca. 600 mg/Kapsel in Kapseln abgefüllt.

In einer Kapsel sind enthalten:
1.) ca. 120 mg Johanniskrautextrakt mit einem Gehalt von ca. 50 mg Hyperforin
2.) ca. 50 mg Kava Kava - Extrakt mit einem Gehalt von ca. 35 mg an Kavapyronen
3.) ca. 40 mg Lavendelextrakt mit einem Gehalt von ca 12 mg Linalool und ca. 13 mg Linalylacetat
4.) ca. 25 mg S-Adenosylmethionin (SAMe)
5.) ca. 50 mg Chia-Samenextrakt mit einem Gehalt von ca. 35 mg alpha-Linolensäure
6.) ca. 150 mg Aminosäurenkomplex und molekulare Mikronährstoffe
7.) ca. 150 mg MCT-Öl (als Trägerstoff)

### 5.1.2 In einer weiteren Ausführungsform wurde statt Kava Kava-Extrakt, 40 mg Rosenwurzpulver (Rhodiola rosea L., ganze Pflanze) mit einem Gehalt von etwa 4% Rosavin und etwa 1% Salidrosid verwendet. Die übrigen Komponenten wurden wie oben verwendet.

### 5.2 Verwendung der Kapseln zur Behandlung von an Depression erkrankter Patienten

Im Rahmen der vorliegenden Erfindung wurde eine erste klinische Studie durchgeführt. Vor der Behandlung wurde der PHQ-9 Score (PHQ-9 Skalensummenwert) der Patienten mittels eines PHQ-9 Depressionserhebungsbogens (ANNEX 1) ermittelt. Teilweise wurden während der Behandlungsdauer auch Zwischenbefunde erhoben.

Die meisten Patienten wurden zuvor bereits schulmedizinisch behandelt (Psychotherapie und z.T. Psychopharmaka-Behandlung), ohne dass sich eine signifikante Besserung der Beschwerden einstellte.

Kurz vor, sowie während der Behandlung mit dem erfindungsgemäßen Kombinationspräparat fand keine Behandlung mit schulmedizinischen Psychopharmaka oder Therapie durch einen Psychotherapeuten statt.

### 5.2.1 Dosierung

Während der Behandlung, die zwischen zwei und drei Wochen dauerte, haben die Patienten ausschließlich das erfindungsgemäße Kombinationspräparat (in Form von Kapseln) eingenommen. Die Konfektionierung der Kapseln war wie in Abschnitt 5.1.1 beschrieben.

Da kein Patient übergewichtig war, war die einheitliche Dosierung: 1. bis 10. Tag: 1 Kapsel morgens, 2 Kapseln mittags, 1 Kapsel abends; ab 11. Tag: 1 Kapsel morgens, 1 Kapsel abends. Eine Kapsel war wie in Abschnitt 5.1.1 konfektioniert.

Den Patienten war freigestellt, bei einer spürbaren Besserung die Einnahme der Kapseln zu reduzieren bzw. zu beenden. So hat die Patientin U.H. (Fallbeispiel 5) die Behandlung nach nur zwei Wochen beendet. Die übrigen Patienten beendeten die Behandlung nach ca. 3 Wochen.

### 5.2.2 Fallbeispiele

### Fallbeispiel 1: Patient: S.M., Geschlecht: männlich, Alter: 55 Jahre

Anamnese: Bei dem Patienten stellte sich vor einigen Monaten plötzlich eine heftige Depression ein, die ihn sofort arbeitsunfähig machte. Er befindet sich seitdem in psychotherapeutischer Behandlung.
Diagnose (nach ICD - 10): Atypische Depression (F 32.8)

Behandlung mit dem Kombinationspräparat: Nach einer zweiwöchigen Behandlung konnte eine teilweise Verbesserung des Zustandes festgestellt werden. Die Phasen der Schlaflosigkeit verringerten sich, damit verbunden auch die Müdigkeit. Die Konzentrationsfähigkeit verstärkte sich. Der mit dem Depressionserhebungsbogen PHQ-9 ermittelte Score des Patienten verbesserte sich von 19 auf 18.

### Fallbeispiel 2: Patient: R.H., Geschlecht: weiblich, Alter: 62 Jahre

Anamnese: Die 62 jährige Frau leidet seit mehr als acht Jahren unter Unruhezuständen mit Depressionen, die eine Reaktion auf äußere Umstände darstellen (Ehezwistigkeiten, unversöhnlicher Streit mit der erwachsenen Tochter u.ä.) Die Patientin befindet sich in Psychotherapie und nimmt seit acht Jahren Psychopharmaka, ohne eine Besserung zu spüren.
Diagnose (nach ICD - 10): Schwere depressive Episode ohne psychotische Symptome (F 32. 2)

Behandlung mit dem Kombinationspräparat: Unmittelbar vor Beginn der Einnahme empfand die Patientin fast täglich eine Niedergeschlagenheit, Schwermut und Hoffnungslosigkeit, Schlaflosigkeit, Gefühle des Versagens und der Energielosigkeit und vor allem Gedanken, lieber tot zu sein oder sich Leid zufügen zu wollen.

Nach zwei Wochen war der Zustand deutlich verbessert; Niedergeschlagenheit, Schwermut und Hoffnungslosigkeit wurden nur noch selten empfunden. Gedanken, lieber tot zu sein oder sich Leid zufügen zu wollen, waren völlig verschwunden. Der mit dem Depressionserhebungsbogen PHQ-9 ermittelte Score der Patientin verbesserte sich von 25 auf 7.

### Fallbeispiel 3: Patient: T.S., Geschlecht: weiblich, Alter: 41 Jahre

Anamnese: Bei der Patientin liegen seit drei Jahren depressive Symptome vor, die sich im Laufe dieser Zeit immer mehr gesteigert haben bis zu heftigen Angst- und Panikzuständen, teilweise sogar mit schweren Halluzinationen. Die Patientin schildert ihre Symptomatik als regelrechten Schockzustand, der sie außerstande setzt, normal zu funktionieren. Seit einem halben Jahr ist die Patientin in psychotherapeutischer Behandlung; während der letzten drei Monate wurde ihr auch - ohne jeglichen Erfolg - ein schulmedizinisches Antidepressivum verabreicht.
Diagnose: (nach ICD - 10): Schwere depressive Episode mit psychotischen Symptomen (F 32.3)

Behandlung mit dem Kombinationspräparat: Bereits eine Woche nach der Einnahme des Kombinationspräparates schildert die Patientin, dass sie keinerlei Panikattacken mehr hat und sich das erste Mal seit langem wieder richtig wohl fühlt. Nach 25 Tagen Therapie mit dem Kombinationspräparat sind die Halluzinationen völlig weg. Die Patientin hat eine vollkommene innere Ruhe und fühlt sich richtig gut. Der mit dem Depressionserhebungsbogen PHQ-9 ermittelte Score der Patientin verbesserte sich von 19 auf 6.

### Fallbeispiel 4: Patient: E.K., Geschlecht: weiblich, Alter: 40 Jahre

Anamnese: Die Patientin ist seit einem Jahr depressiv. Bei ihr sind im Vordergrund Mißlaunigkeit und völlige Schlaflosigkeit, so dass sie ständig "gerädert" ist, sich sehr bedrückt fühlt und somit den ganzen Tag gestresst ist. Seit Beginn ihrer Depression befindet sie sich in Psychotherapie, während der ihr auch schulmedizinische Psychopharmaka verordnet wurden. Hierdurch wurde zwar die Schlaflosigkeit gelindert, jedoch verbunden mit erheblichen Nebenwirkungen wie Müdigkeit am Tage und extreme Gewichtszunahme. Daraufhin hat sie wegen der Nebenwirkungen die Medikamente wieder abgesetzt, worauf sofort die Schlaflosigkeit wieder einsetzte.
Diagnose (nach ICD 10): Mittelgradige depressive Episode (F 32.1)

Behandlung mit dem Kombinationspräparat: Nach einer dreiwöchigen Einnahme berichtet die Patientin, dass es ihr psychisch sehr viel besser geht und sie wieder richtig durchschläft, sich auch wieder nach außen wendet, mit Freunden ausgeht und wieder eine richtige Lebensfreude empfindet. Der mit dem Depressionserhebungsbogen PHQ-9 ermittelte Score der Patientin verbesserte sich von 12 auf 7.

### Fallbeispiel 5: Patient: U.H., Geschlecht: weiblich, Alter: 57 Jahre

Anamnese: Seit vier Jahren liegt bei der Patientin eine schwere Depression vor. Zuletzt hat sie die ganzen Wochenenden im Bett gelegen und konnte sich nicht mehr aufraffen, das Bett zu verlassen. Sie empfand keine Lebensfreude mehr, sondern hat das Wochenende im Bett durchgeweint. Seit drei Jahren ist sie in einer Psychotherapie und bekommt ein schulmedizinisches Antidepressivum, das aber den Zustand nicht verbessert hat.
Diagnose (nach ICD - 10): Schwere depressive Episode ohne psychotische Symptome (F32.2.)

Behandlung mit dem Kombinationspräparat: Nach einer nur zweiwöchigen Therapie mit dem Kombinationspräparat berichtete die Patientin, dass es ihr deutlich besser gehe. Der Ehepartner der Patientin konnte die deutliche Besserung ebenfalls bestätigen, er äußerte sich über den Zustand seiner Frau sogar hellauf begeistert, weil sie seiner Wahrnehmung nach wieder "sehr viel besser drauf" sei. Der mit dem Depressionserhebungsbogen PHQ-9 ermittelte Score der Patientin verbesserte sich durch die nur zweiwöchige Behandlung von 19 auf 12.

### Fallbeispiel 6: Patient: I.T., Geschlecht: weiblich, Alter: 64 Jahre

Anamnese: Die Patientin leidet seit vielen Jahren an einer Depression, die sich vor 15 Jahren stark verschlimmerte, als bei ihr ein Mammakarzinom festgestellt wurde, das in der Folgezeit dann noch rezidivierte, worauf sie in ständiger Angst lebte, mit Schlafstörungen und Depressionen. Die bisherige schulmedizinische Behandlung umfasste Psychotherapie und Behandlung mit Psychopharmaka. Ferner wurden ihr auch, nachdem die schulmedizinische Behandlung nicht anschlug, auch naturheilkundliche Pharmaka (Neurexan) sowie Beruhigungsmittel (Biosedon) verordnet, die aber ebenfalls keine Besserung brachten.
Diagnose (nach ICD - 10): Schwere depressive Episode ohne psychotische Symptome (F 32.2.)

Behandlung mit dem Kombinationspräparat: Nach einer nur zweiwöchigen Behandlung konnte eine geringfügige Besserung festgestellt werden: die tägliche Unfreude an der Arbeit ließ nach sowie auch die Energielosigkeit. Vor der Behandlung hatte die Patientin an mehr als der Hälfte aller Tage das Gefühl zu versagen bzw. ihre Familie zu enttäuschen. Dieses depressive Empfinden verspürte die Patientin nach der Behandlung mit dem Kombipräparat nur noch vereinzelt. Der mit dem Depressionserhebungsbogen PHQ-9 ermittelte Score der Patientin verbesserte sich von 17 auf 14.

### Fallbeispiel 7: Patient: G.M., Geschlecht männlich, Alter: 50 Jahre

Anamnese: Der Patient ist Tierarzt und klagt über viel Stress, da er Tag und Nacht in seinem Dienst verfügbar sein muss. Er neigt dazu, sich schnell aufzuregen und ist außerstande, diesen Zustand zu lindern, worüber er sich selbst wiederum ärgert. Dieser Ärger führt bei dem Patienten zu immer wieder heftigen Depressionen, Minderwertigkeitsgefühlen und absoluter Schlaflosigkeit. Aus zeitlichem Mangel hat der Patient eine psychotherapeutische Behandlung bislang nicht in Anspruch nehmen wollen bzw. können, zumal er auch die Einnahme von Psychopharmaka ablehnt.
Diagnose (nach ICD 10): Leichte depressive Episode (F 32.0.)

Behandlung mit dem Kombinationspräparat: Nach einer zweiwöchigen Behandlung fühlt sich der Patient deutlich besser. Die täglichen Schlafstörungen traten nur noch vereinzelt auf, womit sich auch das Gefühl der Energielosigkeit verbesserte. Auch das Gefühl, ein Versager zu sein und die Familie zu enttäuschen, verschwand völlig. Der mit dem Depressionserhebungsbogen PHQ-9 ermittelte Score des Patienten verbesserte sich von 7 auf 4.

### 5.3 Ergebnisse der Studie

Insgesamt zeigte die Studie eine sehr gute Wirksamkeit des erfindungsgemäßen Kombinationspräparats. Das Präparat wurde gut vertragen, keiner der Patienten berichtete über unerwünschte Nebenwirkungen.

Als in dieser Eindeutigkeit positiv überraschendes Ergebniss der Studie berichteten 85% (6 der 7) der behandelten Personen über eine deutlich von Ihnen wahrgenommene Besserung ihrer Beschwerden. Bei einigen Patienten hatte sich der PHQ-9-Score (Skalensummenwert) sogar halbiert oder mehr als halbiert. Lediglich ein Patient konnte nur eine geringe Linderung der Beschwerden wahrnehmen. Insbesondere wurde bei drei der fünf Patienten, die die Frage i. des PHQ-9 Fragebogens (Annex 1) nicht mit "0" beantwortet hatten, eine signifikante Verbesserung erreicht.

Die Behandlungsdauer betrug im Mittel 20 Tage. Die durchschnittliche Verbesserung des Scores in der klinischen Studie betrug etwa 41% (Tabelle 1). Dies ist insbesondere bemerkenswert, da die meisten Patienten bereits unter psychotherapeutischer Behandlung standen, die den bestehenden Zustand jedoch nicht oder nicht weiter verbessern konnte.

Die Ergebnisse der Gesamtscores (Skalensummenwert) der Patienten sind in Figur 1 abgebildet. Aus Figur 1 (sowie Tabelle 1) wird zudem auch ersichtlich, dass die dimensionale Auswertung der Skalensummenwerte der Patienten (Einschätzung des Schweregrads der Depression) in 6 von 7 Fällen eine Verbesserung der Depressiven Symptomatik durch die Behandlung erreicht wurde. In Zwei Fällen reduzierte sich der Schweregrad der Depression sogar von einer schweren Depression hin zu einer milden Depression, in zwei weiteren Fällen wurde die schwere Symptomatik in eine mittelgradige Symptomatik überführt, in jeweils einem Fall wurde eine mittelgradige Symptomatik in eine leichte Symptomatik überführt und eine leichte Symptomatik in eine minimale Symptomatik (im Prinzip keine behandlungsbedürftige Symptomatik) überführt.

**Tabelle 1**

| PHQ-9 Frage | Patient R.H. | Patient I.T. | Patient U.H. | Patient E.K. | Patient S.M. | Patient T.S. | Patient G.M. |
|---|---|---|---|---|---|---|---|
| a. | 3 / 1 | 3 / 2 | 3 / 2 | 2 / 1 | 3 / 3 | 2 / 1 | 1 / 1 |
| b. | 3 / 1 | 2 / 2 | 2 / 2 | 1 / 1 | 2 / 2 | 2 / 1 | 1 / 1 |
| c. | 3 / 1 | 2 / 2 | 3 / 2 | 3 / 1 | 3 / 2 | 3 / 0 | 3 / 1 |
| d. | 3 / 1 | 3 / 2 | 3 / 2 | 2 / 1 | 3 / 2 | 2 / 1 | 1 / 0 |
| e. | 3 / 1 | 1 / 1 | 2 / 1 | 1 / 0 | 2 / 2 | 2 / 0 | 0 / 0 |
| f. | 3 / 1 | 2 / 1 | 2 / 1 | 1 / 1 | 1 / 3 | 2 / 1 | 1 / 0 |
| g^{.} | 2 / 1 | 2 / 2 | 2 / 2 | 1 / 1 | 3 / 2 | 3 / 1 | 0 / 1 |
| h. | 2 / 1 | 1 / 1 | 1 / 0 | 1 / 1 | 1 / 1 | 2 / 1 | 0 / 0 |
| i. | 3 / 1 | 1 / 1 | 1 / 0 | 0 / 0 | 1 / 1 | 1 / 0 | 0 / 0 |
| Summenwert | 25 / 7 | 17 / 14 | 19 / 12 | 12 / 7 | 19 / 18 | 19 / 6 | 7 / 4 |
| Verbesserung in % | 72 % | 18 % | 37 % | 42 % | 5 % | 68 % | 43 % |
| Verbesserung im Schweregrad | 2 Stufen | 1 Stufe | 1 Stufe | 1 Stufe | - | 2 Stufen | 1 Stufe |
| *Die in der Tabelle angegebenen Werte sind vorher* / *nachher Werte, d.h. der erste Wert ist der vom Patienten für die jeweilige Frage angekreuzte Wert des PHQ-9 Depressionserhebungsbogens vor der Behandlung, der zweite Wert ist der vom Patienten für die jeweilige Frage angekreuzte Wert des PHQ-9 Depressionserhebungsbogens nach Abschluss der Behandlung mit dem Kombinationspräparat* | | | | | | | |

Auffällig ist weiterhin, dass bei allen behandelten Patienten die Müdigkeit oder das Gefühl keine Energie zu haben, reduziert wurde (Frage d. des Fragebogens) und bei 6 von 7 behandelten Patienten ferner überwiegend deutliche Verbesserungen der Einschlaf- oder Durchschlafprobleme (Frage c. des Fragebogens) erreicht wurden.

Insgesamt betrachtet kann dem Kombinationspräparat eine überraschend signifikante Wirksamkeit gegenüber depressiven Symptomatiken zugesprochen werden, die in 85% der bisher untersuchten Fälle zu einer Reduktion im Schweregrad der Depression geführt hat, ohne den Biorhythmus der Patienten negativ zu beeinträchtigen (wie es bei schulmedizinischen Psychopharmaka oft der Fall ist). Ferner wurde auch bei einem frühzeitigen Absetzen der Medikation keine unerwünschten Effekte (Rebound-Phänomene) beobachtet. Weiterhin konnte die Wirksamkeit des Kombinationspräparats verglichen ohne vorangegangene Medikation mit Psychopharmaka, als auch im Vergleich zu einer früheren Behandlung mit natürlichen wie schulmedizinischen Psychopharmaka (4 Probanden) gezeigt werden. Insbesondere auf dem Hintergrund, dass die Patienten zum Teil in bereits langjähriger Behandlung gegen Depressionen waren, sind die mit dem Kombinationspräparat erreichten Ergebnisse als überaschend positiv zu bewerten.

### 5.4 Weitere Ergebnisse

*Kombinationspräparat mit Rosenwurz:* In einer alternativen Konfektionierung wurde die Komponente (ii) der Zusammensetzung als Rosenwurzpulver ausgeführt (wie in Abschnitt 5.1.2 beschrieben). Die bisher erhaltenen Ergebnisse zeigen, dass die anti-depressive Wirkung des Kombinationspräparats aus Abschnitt 5.1.1 beibehalten werden konnte. Die alternative Ausführungsform wurde gestaltet, da KavaKava Extrakte nicht in allen Ländern als Pharmazeutikum freigegeben sind. Sowohl Kava als auch Rhodiola weisen eine angstlösende Wirkung auf und sind somit in der vorliegenden erfindungsgemäßen Zusammensetzung als gleichwertige Alternativen einsetzbar.
*Fallbeispiel:* Der Patient ist ein jungen Biobauer (ca. 35 Jahre alt ca. 95kg). Der Mann ist seit 6 Jahren in Behandlung wegen bipolarer Störungen und völliger Arbeitsunlust. Seit 6 Jahren ging der Mann keinerlei Arbeit mehr nach, war antriebslos, schlief untertags und war nachtaktiv. Trotz zahlreicher vorhergehenden Behandlungsmethoden konnte keinerlei Verbesserung erreicht werden.

Die Behandlung erfolgte mit dem Kombinationspräparat wie in Abschnitt 5.1.2 ausgeführt (Neurophytol), zunächst je eine Kapsel morgens, mittags, abends, für die ersten 2 Wochen. Danach wurde die Dosis auf 2 Kapseln täglich reduziert.

Nach 3 Wochen ging er zum ersten Mal wieder mit seiner Familie aus. Nach 6 Wochen Behandlung teilte der Patient mit, dass er an diesem Tag das erste Mal wieder auf dem Feld arbeitet.

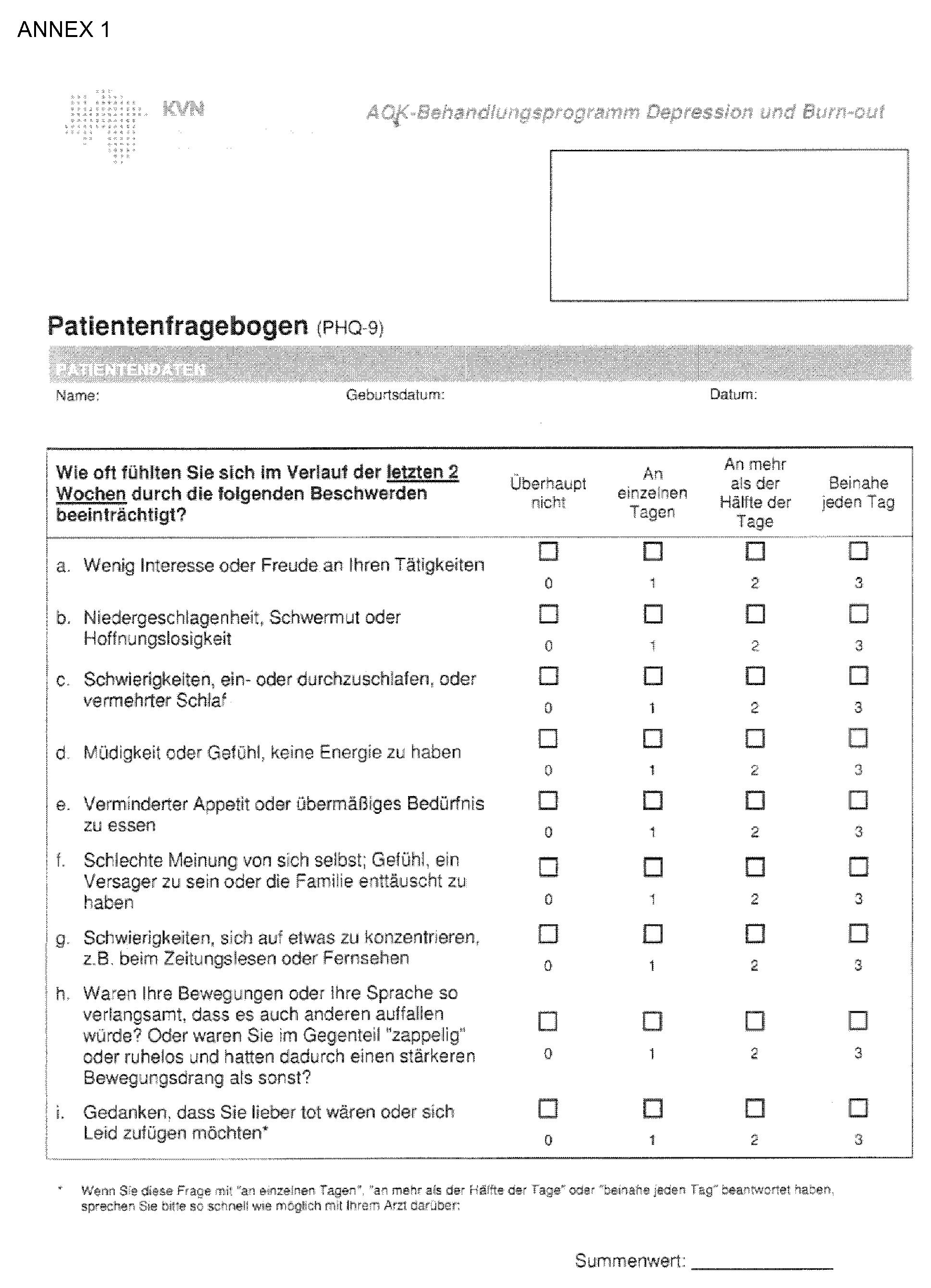

## Patentansprüche

1. Zusammensetzung, umfassend fünf der folgenden Komponenten (i) bis (v):
(i) eine ein oder mehrere Hyperforine enthaltende Komponente,
(ii) eine ein oder mehrere Kavapyrone enthaltende Komponente, oder eine ein oder mehrere Rosavine enthaltende Komponente
(iii) eine Linalool und Linalylacetat enthaltende Komponente,
(iv) eine S-Adenosylmethionin enthaltende Komponente, und
(v) eine Linolsäure und Linolensäure enthaltende Komponente.

2. Zusammensetzung gemäß Anspruch 1, wobei die Zusammensetzung MCT-Öl enthält.

3. Zusammensetzung gemäß Anspruch 1 oder 2, wobei die Zusammensetzung ferner eine L-Dopa enthaltende Komponente, vorzugsweise einen *Mucuna pruriens* Extrakt, umfasst.

4. Zusammensetzung gemäß einem beliebigen der Ansprüche 1 bis 3, wobei die Zusammensetzung einen Aminosäurekomplex enthält, vorzugsweise enthält der Aminosäurekomplex Tryptophan, Glutamin, Tyrosin, Carnitin und Phenylalanin.

5. Zusammensetzung gemäß einem beliebigen der Ansprüche 1 bis 4, ferner umfassend einen oder mehrere Mikronährstoffe ausgewählt aus der Gruppe bestehend aus Vitamin A, B1, B2, B3 (Niacin), B5 (Pantothensäure), B7 (Biotin), B9 (Folsäure), B12, C, D, E, K, Cobalt, Eisen, Fluor, Jod, Kupfer, Mangan, Molybdän, Selen, Silicium, Vanadium, Zink, Zinn, Arsen, Bor und Rubidium, vorzugsweise umfasst die pharmazeutische Zusammensetzung Vitamin B-Komplex (Vitamine B1, B2, B6, B12, Biotin, Niacin und Pantothensäure), Folsäure und L-Carnitin.

6. Zusammensetzung gemäß einem beliebigen der Ansprüche 1 bis 5, wobei die Komponenten (i), (ii), (iii) und (v) Pflanzenextrakte sind.

7. Zusammensetzung gemäß einem beliebigen der Ansprüche 1-6, wobei die Komponente (i) ausgewählt ist aus einem Pflanzenextrakt aus den Zweigspitzen oder Blüten des *Hypericum perforatum* oder *Hypericum calycinum,* oder einem Pflanzenextrakt, der einen Hyperforin-Anteil von wenigstens 15% (w/w) aufweist, die Komponente (ii) ausgewählt ist aus einem Pflanzenextrakt aus der Wurzel des *Piper methysticum,* einem Kava-Kavawurzelextrakt, oder einem Pflanzenextrakt, der einen Kavapyron-Anteil von wenigstens 30% (w/w) aufweist oder die Rosavarine enthaltende Komponente ein Extrakt aus Rosenwurz ist, der einen Rosavarin-Anteil von wenigstens 1% (w/w) aufweist ist, die Komponente (iii) ausgewählt ist aus ein Pflanzenextrakt aus den Blüten des *Lavandula officinalis,* oder aus einem Extrakt aus *Salvia sclarea,* aus *Citrus bergamia,* Koriander, Hopfen, Muskat, Ingwer, Bohnenkraut, Zimt, Basilikum, Majoran, Thymian, Oregano, schwarzem Pfeffer oder Safran, oder einem Pflanzenextrakt, der einen Linalool/Linalylacetat-Anteil von wenigstens 40% (w/w) aufweist, die Komponente (iv) ausgewählt ist aus S-Adenosylmethionin oder einer Kombination des reinen Stoffs S-Adenosylmethionin mit einem oder weiteren Stoffen, und die Komponente (v) ausgewählt ist aus einem Pflanzenextrakt aus Samen des *Salvia hispanica,* Chiasamen-Öl, Traubenkernöl, Distelöl, Safloröl, Hanföl, Sojaöl, Baumwollsaatöl, Weizenkeimöl, Maiskeimöl, Sonnenblumenöl und Rapsöl, oder einem anderen Pflanzenextrakt mit einem Linolsäure/alpha-Linolensäure Anteil von wenigstens 40% (w/w) wobei die Auswahl einer jeweiligen Komponente unabhängig von der Auswahl der anderen Komponenten ist.

8. Zusammensetzung gemäß einem beliebigen der Ansprüche 1-7, wobei die Zusammensetzung einen Gehalt von 1-1000 mg Hyperforine, 1-1000mg Kavapyrone oder 0,1-20 mg Rosavarine, 0,5-1000mg Linalool/Linalylacetat, 0,15-1000 mg S-Adenosylmethionin und 1-1000 mg Linolsäure/Linolensäure aufweist, wobei der Gehalt einer jeweiligen Komponente unabhängig von dem Gehalt der anderen Komponenten ist.

9. Zusammensetzung gemäß einem beliebigen der Ansprüche 1-8 zur Verwendung bei der Behandlung einer depressiven Störung.

10. Zusammensetzung zur Verwendung gemäß Anspruch 9, wobei die depressive Störung ausgewählt ist aus einer leichten, mittelgradigen oder schweren depressive Episode, psychogener Depression, reaktiver Depression, rezidivierender depressiver Störung, anhaltender affektiver Störung, Zyklothymia, bipolarer affektiver Störung, Dysthymia, und bipolarer Störungen, wie Bipolar-I Störung und Bipolar-II-Störung.

11. Zusammensetzung zur Verwendung gemäß einem Beliebigen der Ansprüche 9 bis 10, wobei die Zusammensetzung in einer Dosierung von (i) 0,1-20 mg/kg Körpergewicht/Tag Hyperforine, (ii) 0,1-15 mg/kg Körpergewicht/Tag Kavapyrone oder 5-1000 µg/kg Körpergewicht/Tag Rosavarine, (iii) 0,1-15 mg/kg Körpergewicht/Tag Linalool/Linalylacetat, (iv) 0,1-15 mg/kg Körpergewicht/Tag SAMe, und (v) 0,1-15 mg/kg Körpergewicht/Tag Linolsäure/Linolensäure zu verabreichen ist, wobei die Dosierung einer jeweiligen Komponente unabhängig von der Dosierung der anderen Komponenten ist.

12. Zusammensetzung nach einem beliebigen der Ansprüche 1 bis 8, wobei die Zusammensetzung eine pharmazeutische Zusammensetzung ist.

13. Zusammensetzung nach einem beliebigen der Ansprüche 1 bis 8, wobei die Zusammensetzung als Nahrungsergänzungsmittel ausgeführt ist.

14. Verfahren zur Herstellung einer pharmazeutischen Zusammensetzung, umfassend
(1) das Bereitstellen
(i) einer ein oder mehrere Hyperforine enthaltenden Komponente,
(ii) einer ein oder mehrere Kavapyrone enthaltenden Komponente oder einer ein oder mehrere Rosavine enthaltenden Komponente,
(iii) einer Linalool und Linalylacetat enthaltenden Komponente,
(iv) einer S-Adenosylmethionin enthaltenden Komponente, und
(v) einer Omega-3 und Omega-6 Fettsäuren enthaltenden Komponente,
(2) Formulieren der Komponenten aus (1) in Form einer Tablette, Kapsel, Pille, Pastille oder eines Pulvers, einer essbaren Zusammensetzung, eines Nahrungsergänzungsmittels oder eines Getränks.

## Claims

1. Composition comprising five of the following components (i) to (v):
(i) one or more components containing hyperforin,
(ii) one or more components containing kavapyrone, or one or more components containing rosavin,
(iii) one component containing linalool and linalyl acetate,
(iv) one component containing S-adenosyl methionine, and
(v) one component containing linoleic acid and linolenic acid.

2. Composition according to claim 1, wherein the composition contains MCT oil.

3. Composition according to either claim 1 or claim 2, wherein the composition also comprises a component, preferably an extract of *Mucuna pruriens,* containing L-Dopa.

4. Composition according to any of claims 1 to 3, wherein the composition contains an amino acid complex, preferably contains the amino acid complex tryptophan, glutamine, tyrosine, carnitine and phenylalanine.

5. Composition according to any of claims 1 to 4, also comprising one or more micronutrients selected from the group consisting of vitamins A, B1, B2, B3 (niacin), B5 (pantothenic acid), B7 (biotin), B9 (folic acid), B12, C, D, E and K, cobalt, iron, fluorine, iodine, copper, manganese, molybdenum, selenium, silicon, vanadium, zinc, tin, arsenic, boron and rubidium, the pharmaceutical composition preferably comprising vitamin B complex (vitamins B1, B2, B6, B12, biotin, niacin and pantothenic acid), folic acid and I-carnitine.

6. Composition according to any of claims 1 to 5, wherein the components (i), (ii), (iii) and (v) are plant extracts.

7. Composition according to any of claims 1 to 6, wherein the component (i) is selected from a plant extract from the branch tips or flowers of *Hypericum perforatum* or *Hypericum calycinum,* or a plant extract comprising a proportion of hyperforin of at least 15% (w/w); the component (ii) is selected from a plant extract from the root of *Piper methysticum,* a kava-kava root extract or a plant extract which has a proportion of kavapyrone of at least 30% (w/w), or the component containing rosavin is an extract of *Rhodiola rosea* comprising a proportion of rosavin of at least 1% (w/w); the component (iii) is selected from a plant extract from the flowers of *Lavandula officinalis,* an extract of *Salvia sclarea, Citrus bergamia,* coriander, hops, nutmeg, ginger, *Satureja,* cinnamon, basil, marjoram, thyme, oregano, black pepper or saffron, or a plant extract comprising a proportion of linalool/linalyl acetate of at least 40% (w/w); the component (iv) is selected from S-adenosyl methionine or a combination of the pure substance S-adenosyl methionine and one or more substances; and the component (v) is selected from a plant extract of *Salvia hispanica* seeds, chia seed oil, grapeseed oil, thistle oil, safflower oil, hemp oil, soybean oil, cottonseed oil, wheatgerm oil, corn oil, sunflower oil and rapeseed oil, or a different plant extract comprising a proportion of linoleic acid/alpha-linolenic acid of at least 40% (w/w), wherein each component is selected independently of the other components.

8. Composition according to any of claims 1 to 7, wherein the composition comprises a content of 1-1000 mg hyperforin, 1-1000 mg kavapyrone or 0.1-20 mg rosavin, 0.5-1000 mg linalool/linalyl acetate, 0.15-1000 mg S-adenosyl methionine and 1-1000 mg linoleic acid/ linolenic acid, wherein the content of each component is independent of the contents of the other components.

9. Composition according to any of claims 1 to 8 for use in the treatment of a depressive disorder.

10. Composition for use according to claim 9, wherein the depressive disorder can be a mild, moderate or severe depressive episode, psychogenic depression, reactive depression, recurring depressive disorder, long-term affective disorder, cyclothymia, bipolar affective disorder, dysthymia and bipolar disorders such as bipolar I disorder and bipolar II disorder.

11. Composition for use according to either claim 9 or claim 10, wherein the composition is to be administered in a dose of (i) 0.1-20 mg/kg of body weight per day of hyperforin, (ii) 0.1-15 mg/kg of body weight per day of kavapyrone or 5-1000 µg/kg of body weight per day of rosavin, (iii) 0.1-15 mg/kg of body weight per day of linalool/linalyl acetate, (iv) 0.1-15 mg/kg of body weight per day of SAMe, and (v) 0.1-15 mg/kg of body weight per day of linoleic acid/ linolenic acid, wherein the dose of each component is independent of the dose of the other components.

12. Composition according to any of claims 1 to 8, wherein the composition is a pharmaceutical composition.

13. Composition according to any of claims 1 to 8, wherein the composition is formed as a dietary supplement.

14. Method for preparing a pharmaceutical composition, comprising
(1) providing
(i) one or more components containing hyperforin,
(ii) one or more components containing kavapyrone or one or more components containing rosavin,
(iii) one component containing linalool and linalyl acetate,
(iv) one component containing S-adenosyl methionine, and
(v) one component containing omega-3 and omega-6 fatty acids,
(2) formulating the components from (1) into the form of a tablet, capsule, pill, pastille or powder, an edible composition, a dietary supplement or a beverage.

## Revendications

1. Composition, comprenant cinq des composants (i) à (v) suivants :
(i) un composant contenant une ou plusieurs hyperforines,
(ii) un composant contenant une ou plusieurs kavapyrones, ou un composant contenant une ou plusieurs rosavines,
(iii) un composant contenant du linalool et de l'acétate de linalyle,
(iv) un composant contenant de la S-adénosylméthionine et
(v) un composant contenant de l'acide linoléique et de l'acide linolénique.

2. Composition selon la revendication 1, dans laquelle la composition contient de l'huile de TCM.

3. Composition selon la revendication 1 ou 2, dans laquelle la composition comprend en outre un composant contenant de la L-Dopa, de préférence un extrait de *Mucuna pruriens.*

4. Composition selon l'une quelconque des revendications 1 à 3, dans laquelle la composition contient un complexe d'acide aminé, le complexe d'acide aminé contenant de préférence du tryptophane, de la glutamine, de la tyrosine, de la carnitine et de la phénylalanine.

5. Composition selon l'une quelconque des revendications 1 à 4, comprenant en outre un ou plusieurs micronutriments choisis dans le groupe constitué par la vitamine A, B1, B2, B3 (niacine), B5 (acide pantothénique), B7 (biotine), B9 (acide folique), B12, C, D, E, K, le cobalt, le fer, le fluor, l'iode, le cuivre, le manganèse, le molybdène, le sélénium, le silicium, le vanadium, le zinc, l'étain, l'arsenic, le bore et le rubidium, la composition pharmaceutique comprenant de préférence un complexe de vitamines B (vitamines B1, B2, B6, B12, biotine, niacine et acide pantothénique), de l'acide folique et de la L-carnitine.

6. Composition selon l'une quelconque des revendications 1 à 5, dans laquelle les composants (i), (ii), (iii) et (v) sont des extraits végétaux.

7. Composition selon l'une quelconque des revendications 1 à 6, dans laquelle le composant (i) est choisi parmi un extrait végétal des pointes de branches ou des fleurs d'*Hypericum perforatum* ou d'*Hypericum calycinum,* ou un extrait végétal qui présente une proportion d'hyperforine d'au moins 15 % (m/m), le composant (ii) est choisi parmi un extrait végétal de la racine de *Piper methysticum,* un extrait de racine de kava, ou un extrait végétal qui présente une proportion de kavapyrone d'au moins 30 % (m/m), ou le composant contenant des rosavarines est un extrait d'orpin rose, qui présente une proportion de rosavarine d'au moins 1 % (m/m), le composant (iii) est choisi parmi un extrait végétal des fleurs de *Lavandula officinalis,* ou un extrait de *Salvia sclarea,* de *Citrus bergamia,* de coriandre, de houblon, de muscade, de gingembre, de sarriette, de cannelle, de basilic, de marjolaine, de thym, d'origan, de poivre noir ou de safran, ou un extrait végétal qui présente une proportion de linalool/acétate de linalyle d'au moins 40 % (m/m), le composant (iv) est choisi parmi la S-adénosylméthionine ou une combinaison de la substance pure S-adénosylméthionine avec une ou plusieurs substances, et le composant (v) est choisi parmi un extrait végétal de graines de *Salvia hispanica*, d'huile de graines de chia, d'huile de pépins de raisin, d'huile de chardon, d'huile de carthame, d'huile de chanvre, d'huile de soja, d'huile de graines de coton, d'huile de germes de blé, d'huile de germes de maïs, d'huile de tournesol et d'huile de colza, ou un autre extrait végétal ayant une proportion d'acide linoléique/acide alpha-linolénique d'au moins 40 % (m/m), le choix d'un composant respectif étant indépendant du choix des autres composants.

8. Composition selon l'une quelconque des revendications 1 à 7, dans laquelle la composition présente une teneur de 1 à 1 000 mg en hyperforines, de 1 à 1 000 mg en kavapyrones ou de 0,1 à 20 mg en rosavarines, de 0,5 à 1 000 mg en linalool/acétate de linalyle, de 0,15 à 1 000 mg en S-adénosylméthionine et de 1 à 1 000 mg en acide linoléique/acide linolénique, la teneur d'un composant respectif étant indépendante de la teneur des autres composants.

9. Composition selon l'une quelconque des revendications 1 à 8, destinée à une utilisation dans le traitement d'un trouble dépressif.

10. Composition destinée à une utilisation selon la revendication 9, dans laquelle le trouble dépressif est choisi parmi un épisode dépressif léger, moyen ou sévère, une dépression psychogène, une dépression réactionnelle, un trouble dépressif récidivant, un trouble affectif persistant, une cyclothymie, un trouble affectif bipolaire, une dysthymie et des troubles bipolaires, tels que le trouble bipolaire de type I et le trouble bipolaire de type II.

11. Composition destinée à une utilisation selon l'une quelconque des revendications 9 à 10, dans laquelle la composition est à administrer en un dosage de (i) 0,1 à 20 mg/kg de poids corporel/jour d'hyperforines, (ii) 0,1 à 15 mg/kg de poids corporel/jour de kavapyrones ou 5 à 1 000 µg/kg de poids corporel/jour de rosavarines, (iii) 0,1 à 15 mg/kg de poids corporel/jour de linalool/acétate de linalyle, (iv) 0,1 à 15 mg/kg de poids corporel/jour de SAMe et (v) 0,1 à 15 mg/kg de poids corporel/jour d'acide linoléique/acide linolénique, le dosage d'un composant respectif étant indépendant du dosage des autres composants.

12. Composition selon l'une quelconque des revendications 1 à 8, dans laquelle la composition est une composition pharmaceutique.

13. Composition selon l'une quelconque des revendications 1 à 8, dans laquelle la composition est configurée sous la forme d'un complément alimentaire.

14. Procédé de fabrication d'une composition pharmaceutique, comprenant :
(1) la préparation
(i) d'un composant contenant une ou plusieurs hyperforines,
(ii) d'un composant contenant une ou plusieurs kavapyrones, ou d'un composant contenant une ou plusieurs rosavines,
(iii) d'un composant contenant du linalool et de l'acétate de linalyle,
(iv) d'un composant contenant de la S-adénosylméthionine et
(v) d'un composant contenant des acides gras oméga 3 et oméga 6,
(2) la formulation des composants de (1) sous la forme d'un comprimé, d'une gélule, d'une pilule, d'une pastille ou d'une poudre, d'une composition comestible, d'un complément alimentaire ou d'une boisson.
